# EUROPÄISCHE PATENTSCHRIFT

(11) **EP 2 036 979 B1**
(45) Veröffentlichungstag und Bekanntmachung des Hinweises auf die Patenterteilung: **26.10.2016**
(21) Anmeldenummer: 08162381.1
(22) Anmeldetag: 14.08.2008
(51) Int. Cl.: C12N 15/61, C12N 9/90, C12P 13/08, C12P 13/22

(54) **Verfahren zur Herstellung von L-Aminosäuren unter Verwendung von verbesserten Stämmen der Familie Enterobacteriaceae**
Method for manufacturing L-amino acids using improved strains of the enterobacteriaceae family
Procédé de fabrication d'aminoacides L en utilisant des troncs améliorés de la famille des enterobacteriaceae

(30) Priorität: 15.09.2007 DE 102007044134
(43) Veröffentlichungstag der Anmeldung: 18.03.2009
(73) Patentinhaber: Evonik Degussa GmbH, 45128 Essen (DE)
(72) Erfinder: Rieping, Mechthild, Dr., 33619 Bielefeld (DE); Kreutzer, Caroline, 33813 Oerlinghausen (DE)

(56) Entgegenhaltungen:
- EP-A- 0 979 876
- EP-A- 1 013 765
- EP-A- 1 016 724
- EP-A- 1 715 055
- WO-A-03/008607
- US-A- 4 278 765
- US-A1- 2007 054 381
- GUTHRIE B ET AL: "Trigger factor depletion or overproduction causes defective cell division but does not block protein export." JOURNAL OF BACTERIOLOGY, Bd. 172, Nr. 10, Oktober 1990 (1990-10), Seiten 5555-5562, XP009006868 ISSN: 0021-9193
- NISHIHARA KAZUYO ET AL: "Overexpression of Trigger Factor prevent aggregation of recombinant proteins in Escherichia coli" APPLIED AND ENVIRONMENTAL MICROBIOLOGY, Bd. 66, Nr. 3, 1. März 2000 (2000-03-01), Seiten 884-889, XP002156141 ISSN: 0099-2240
- KANDROR O ET AL: "Trigger factor is induced upon cold shock and enhances viability of Escherichia coli at low temperatures" PROCEEDINGS OF THE NATIONAL ACADEMY OF SCIENCES OF USA, Bd. 94, 1. Mai 1997 (1997-05-01), Seiten 4978-4981, XP002991343 ISSN: 0027-8424
- HANSMEIER NICOLE ET AL: "The cytosolic, cell surface and extracellular proteomes of the biotechnologically important soil bacterium Cornyebacterium efficiens YS-314 in comparison to those of Corynebacterium glutamicum ATCC 13032" PROTEOMICS, Bd. 6, Nr. 1, Januar 2006 (2006-01), Seiten 233-250, XP002506671 ISSN: 1615-9853
- MAIER T ET AL: "A cradle for new proteins: trigger factor at the ribosome" CURRENT OPINION IN STRUCTURAL BIOLOGY, Bd. 15, Nr. 2, 1. April 2005 (2005-04-01), Seiten 204-212, XP004902397 ISSN: 0959-440X
- HARTL F U ET AL: "Molecular chaperones in the cytosol: From nascent chain to folded protein" SCIENCE, Bd. 295, Nr. 5561, 8. März 2002 (2002-03-08), Seiten 1852-1858, XP002506672 ISSN: 0036-8075
- DEBABOV V G: "THE THREONINE STORY" ADVANCES IN BIOCHEMICAL ENGINEERING, BIOTECHNOLOGY, SPRINGER, BERLIN, DE, Bd. 79, 1. Januar 2003 (2003-01-01), Seiten 113-136, XP008014933 ISSN: 0724-6145

### SEQUENZPROTOKOLL

<110> Evonik Degussa GmbH
<120> Verfahren zur Herstellung von L-Aminosäuren unter Verwendung von verbesserten Stämmen der Familie Enterobacteriaceae
<130> 200700616 AL
<160> 14
<170> Patent In version 3.3
<210> 1
   <211> 1299
   <212> DNA
   <213> Escherichia coli
<220>
   <221> CDS
   <222> (1)..(1299)
   <223> tig Kodierregion
<400> 1
   atg caa gtt tca gtt gaa acc act caa ggc ctt ggc cgc cgt gta acg       48
   Met Gln Val Ser Val Glu Thr Thr Gln Gly Leu Gly Arg Arg Val Thr
   1               5                   10                  15
   att act atc gct gct gac agc atc gag acc gct gtt aaa agc gag ctg       96
   Ile Thr Ile Ala Ala Asp Ser Ile Glu Thr Ala Val Lys Ser Glu Leu
               20                  25                  30
   gtc aac gtt gcg aaa aaa gta cgt att gac ggc ttc cgc aaa ggc aaa      144
   Val Asn Val Ala Lys Lys Val Arg Ile Asp Gly Phe Arg Lys Gly Lys
           35                  40                  45
   gtg cca atg aat atc gtt gct cag cgt tat ggc gcg tct gta cgc cag      192
   Val Pro Met Asn Ile Val Ala Gln Arg Tyr Gly Ala Ser Val Arg Gln
       50                  55                  60
   gac gtt ctg ggt gac ctg atg agc cgt aac ttc att gac gcc atc att      240
   Asp Val Leu Gly Asp Leu Met Ser Arg Asn Phe Ile Asp Ala Ile Ile
   65                  70                  75                  80
   aaa gaa aaa atc aat ccg gct ggc gca ccg act tat gtt ccg ggc gaa      288
   Lys Glu Lys Ile Asn Pro Ala Gly Ala Pro Thr Tyr Val Pro Gly Glu
                   85                  90                  95
   tac aag ctg ggt gaa gac ttc act tac tct gta gag ttt gaa gtt tat      336
   Tyr Lys Leu Gly Glu Asp Phe Thr Tyr Ser Val Glu Phe Glu Val Tyr
               100                 105                 110
   ccg gaa gtt gaa ctg cag ggt ctg gaa gcg atc gaa gtt gaa aaa ccg      384
   Pro Glu Val Glu Leu Gln Gly Leu Glu Ala Ile Glu Val Glu Lys Pro
           115                 120                 125
   atc gtt gaa gtg acc gac gct gac gtt gac ggc atg ctg gat act ctg      432
   Ile Val Glu Val Thr Asp Ala Asp Val Asp Gly Met Leu Asp Thr Leu
       130                 135                 140
   cgt aaa cag cag gcg acc tgg aaa gaa aaa gac ggc gct gtt gaa gca      480
   Arg Lys Gln Gln Ala Thr Trp Lys Glu Lys Asp Gly Ala Val Glu Ala
   145                 150                 155                 160
   gaa gac cgc gta acc atc gac ttc acc ggt tct gta gac ggc gaa gag      528
   Glu Asp Arg Val Thr Ile Asp Phe Thr Gly Ser Val Asp Gly Glu Glu
                   165                 170                 175
   ttc gaa ggc ggt aaa gcg tct gat ttc gta ctg gcg atg ggc cag ggt      576
   Phe Glu Gly Gly Lys Ala Ser Asp Phe Val Leu Ala Met Gly Gln Gly
               180                 185                 190
   cgt atg atc ccg ggc ttt gaa gac ggt atc aaa ggc cac aaa gct ggc      624
   Arg Met Ile Pro Gly Phe Glu Asp Gly Ile Lys Gly His Lys Ala Gly
           195                 200                 205
   gaa gag ttc acc atc gac gtg acc ttc ccg gaa gaa tac cac gca gaa      672
   Glu Glu Phe Thr Ile Asp Val Thr Phe Pro Glu Glu Tyr His Ala Glu
       210                 215                 220
   aac ctg aaa ggt aaa gca gcg aaa ttc gct atc aac ctg aag aaa gtt      720
   Asn Leu Lys Gly Lys Ala Ala Lys Phe Ala Ile Asn Leu Lys Lys Val
   225                 230                 235                 240
   gaa gag cgt gaa ctg ccg gaa ctg act gca gaa ttc atc aaa cgt ttc      768
   Glu Glu Arg Glu Leu Pro Glu Leu Thr Ala Glu Phe Ile Lys Arg Phe
                   245                 250                 255
   ggc gtt gaa gat ggt tcc gta gaa ggt ctg cgc gct gaa gtg cgt aaa      816
   Gly Val Glu Asp Gly Ser Val Glu Gly Leu Arg Ala Glu Val Arg Lys
               260                 265                 270
   aac atg gag cgc gag ctg aag agc gcc atc cgt aac cgc gtt aag tct      864
   Asn Met Glu Arg Glu Leu Lys Ser Ala Ile Arg Asn Arg Val Lys Ser
           275                 280                 285
   cag gcg atc gaa ggt ctg gta aaa gct aac gac atc gac gta ccg gct      912
   Gln Ala Ile Glu Gly Leu Val Lys Ala Asn Asp Ile Asp Val Pro Ala
       290                 295                 300
   gcg ctg atc gac agc gaa atc gac gtt ctg cgt cgc cag gct gca cag      960
   Ala Leu Ile Asp Ser Glu Ile Asp Val Leu Arg Arg Gln Ala Ala Gln
   305                 310                 315                 320
   cgt ttc ggt ggc aac gaa aaa caa gct ctg gaa ctg ccg cgc gaa ctg     1008
   Arg Phe Gly Gly Asn Glu Lys Gln Ala Leu Glu Leu Pro Arg Glu Leu
                   325                 330                 335
   ttc gaa gaa cag gct aaa cgc cgc gta gtt gtt ggc ctg ctg ctg ggc     1056
   Phe Glu Glu Gln Ala Lys Arg Arg Val Val Val Gly Leu Leu Leu Gly
               340                 345                 350
   gaa gtt atc cgc acc aac gag ctg aaa gct gac gaa gag cgc gtg aaa     1104
   Glu Val Ile Arg Thr Asn Glu Leu Lys Ala Asp Glu Glu Arg Val Lys
           355                 360                 365
   ggc ctg atc gaa gag atg gct tct gcg tac gaa gat ccg aaa gaa gtt     1152
   Gly Leu Ile Glu Glu Met Ala Ser Ala Tyr Glu Asp Pro Lys Glu Val
       370                 375                 380
   atc gag ttc tac agc aaa aac aaa gaa ctg atg gac aac atg cgc aat     1200
   Ile Glu Phe Tyr Ser Lys Asn Lys Glu Leu Met Asp Asn Met Arg Asn
   385                 390                 395                 400
   gtt gct ctg gaa gaa cag gct gtt gaa gct gta ctg gcg aaa gcg aaa     1248
   Val Ala Leu Glu Glu Gln Ala Val Glu Ala Val Leu Ala Lys Ala Lys
                   405                 410                 415
   gtg act gaa aaa gaa acc act ttc aac gag ctg atg aac cag cag gcg     1296
   Val Thr Glu Lys Glu Thr Thr Phe Asn Glu Leu Met Asn Gln Gln Ala
               420                 425                 430
   taa                                                                 1299
<210> 2
   <211> 432
   <212> PRT
   <213> Escherichia coli
<400> 2
   Met Gln Val Ser Val Glu Thr Thr Gln Gly Leu Gly Arg Arg Val Thr
   1               5                   10                  15
   Ile Thr Ile Ala Ala Asp Ser Ile Glu Thr Ala Val Lys Ser Glu Leu
               20                  25                  30
   Val Asn Val Ala Lys Lys Val Arg Ile Asp Gly Phe Arg Lys Gly Lys
           35                  40                  45
   Val Pro Met Asn Ile Val Ala Gln Arg Tyr Gly Ala Ser Val Arg Gln
       50                  55                  60
   Asp Val Leu Gly Asp Leu Met Ser Arg Asn Phe Ile Asp Ala Ile Ile
   65                  70                  75                  80
   Lys Glu Lys Ile Asn Pro Ala Gly Ala Pro Thr Tyr Val Pro Gly Glu
                   85                  90                  95
   Tyr Lys Leu Gly Glu Asp Phe Thr Tyr Ser Val Glu Phe Glu Val Tyr
               100                 105                 110
   Pro Glu Val Glu Leu Gln Gly Leu Glu Ala Ile Glu Val Glu Lys Pro
           115                 120                 125
   Ile Val Glu Val Thr Asp Ala Asp Val Asp Gly Met Leu Asp Thr Leu
       130                 135                 140
   Arg Lys Gln Gln Ala Thr Trp Lys Glu Lys Asp Gly Ala Val Glu Ala
   145                 150                 155                 160
   Glu Asp Arg Val Thr Ile Asp Phe Thr Gly Ser Val Asp Gly Glu Glu
                   165                 170                 175
   Phe Glu Gly Gly Lys Ala Ser Asp Phe Val Leu Ala Met Gly Gln Gly
               180                 185                 190
   Arg Met Ile Pro Gly Phe Glu Asp Gly Ile Lys Gly His Lys Ala Gly
           195                 200                 205
   Glu Glu Phe Thr Ile Asp Val Thr Phe Pro Glu Glu Tyr His Ala Glu
       210                 215                 220
   Asn Leu Lys Gly Lys Ala Ala Lys Phe Ala Ile Asn Leu Lys Lys Val
   225                 230                 235                 240
   Glu Glu Arg Glu Leu Pro Glu Leu Thr Ala Glu Phe Ile Lys Arg Phe
                   245                 250                 255
   Gly Val Glu Asp Gly Ser Val Glu Gly Leu Arg Ala Glu Val Arg Lys
               260                 265                 270
   Asn Met Glu Arg Glu Leu Lys Ser Ala Ile Arg Asn Arg Val Lys Ser
           275                 280                 285
   Gln Ala Ile Glu Gly Leu Val Lys Ala Asn Asp Ile Asp Val Pro Ala
       290                 295                 300
   Ala Leu Ile Asp Ser Glu Ile Asp Val Leu Arg Arg Gln Ala Ala Gln
   305                 310                 315                 320
   Arg Phe Gly Gly Asn Glu Lys Gln Ala Leu Glu Leu Pro Arg Glu Leu
                   325                 330                 335
   Phe Glu Glu Gln Ala Lys Arg Arg Val Val Val Gly Leu Leu Leu Gly
               340                 345                 350
   Glu Val Ile Arg Thr Asn Glu Leu Lys Ala Asp Glu Glu Arg Val Lys
           355                 360                 365
   Gly Leu Ile Glu Glu Met Ala Ser Ala Tyr Glu Asp Pro Lys Glu Val
       370                 375                 380
   Ile Glu Phe Tyr Ser Lys Asn Lys Glu Leu Met Asp Asn Met Arg Asn
   385                 390                 395                 400
   Val Ala Leu Glu Glu Gln Ala Val Glu Ala Val Leu Ala Lys Ala Lys
                   405                 410                 415
   Val Thr Glu Lys Glu Thr Thr Phe Asn Glu Leu Met Asn Gln Gln Ala
               420                 425                 430
<210> 3
   <211> 1299
   <212> DNA
   <213> Salmonella enterica
<220>
   <221> CDS
   <222> (1)..(1299)
   <223> tig Kodierregion
<400> 3
   atg caa gtt tca gtt gaa acc act cag ggc ctt ggc cgc cgt gta acg        48
   Met Gln Val Ser Val Glu Thr Thr Gln Gly Leu Gly Arg Arg Val Thr
   1               5                   10                  15
   att aca atc gct gct gac agc atc gag acc gct gta aaa agc gag ctg        96
   Ile Thr Ile Ala Ala Asp Ser Ile Glu Thr Ala Val Lys Ser Glu Leu
               20                  25                  30
   gtc aac gta gcg aaa aaa gta cgt att gac ggc ttc cgt aaa ggc aaa       144
   Val Asn Val Ala Lys Lys Val Arg Ile Asp Gly Phe Arg Lys Gly Lys
           35                  40                  45
   gta ccg atg aat atc gtc gct cag cgt tat ggc gct tct gtt cgc cag       192
   Val Pro Met Asn Ile Val Ala Gln Arg Tyr Gly Ala Ser Val Arg Gln
       50                  55                  60
   gac gtg ctg ggc gat ctg atg agc cgc aac ttc gtt gac gcg atc atc      240
   Asp Val Leu Gly Asp Leu Met Ser Arg Asn Phe Val Asp Ala Ile Ile
   65                  70                  75                  80
   aaa gaa aaa att aac ccg gca ggc gcg ccg aac tat gtt ccg ggc gaa      288
   Lys Glu Lys Ile Asn Pro Ala Gly Ala Pro Asn Tyr Val Pro Gly Glu
                   85                  90                  95
   tac aaa gtg ggt gaa gac ttc act tac tcc gtc gag ttt gaa gtc tat      336
   Tyr Lys Val Gly Glu Asp Phe Thr Tyr Ser Val Glu Phe Glu Val Tyr
               100                 105                 110
   ccg gaa gtt gag ctg act ggc ctc gaa tcc atc gaa gtt gaa aag ccg      384
   Pro Glu Val Glu Leu Thr Gly Leu Glu Ser Ile Glu Val Glu Lys Pro
           115                 120                 125
   gtt gtt gaa gtc acc gac gct gac gtt gat gtg atg ctg gac acc ctg      432
   Val Val Glu Val Thr Asp Ala Asp Val Asp Val Met Leu Asp Thr Leu
       130                 135                 140
   cgt aag cag cag gcg acc tgg aaa gaa aaa gac ggc gct gcg gat gcg      480
   Arg Lys Gln Gln Ala Thr Trp Lys Glu Lys Asp Gly Ala Ala Asp Ala
   145                 150                 155                 160
   gaa gac cgc gtg acc atc gat ttc acc ggc tct gta gac ggc gaa gag      528
   Glu Asp Arg Val Thr Ile Asp Phe Thr Gly Ser Val Asp Gly Glu Glu
                   165                 170                 175
   ttc gaa ggc ggc aaa gcg acc gat ttc gtt ctg gcg atg ggc cag ggt      576
   Phe Glu Gly Gly Lys Ala Thr Asp Phe Val Leu Ala Met Gly Gln Gly
               180                 185                 190
   cgt atg att ccg ggc ttt gaa gac ggc gtt aaa ggc cac aaa gct ggc      624
   Arg Met Ile Pro Gly Phe Glu Asp Gly Val Lys Gly His Lys Ala Gly
           195                 200                 205
   gaa gag ttc act atc gat gtg acc ttc ccg gaa gag tac cac gct gaa      672
   Glu Glu Phe Thr Ile Asp Val Thr Phe Pro Glu Glu Tyr His Ala Glu
       210                 215                 220
   aac ctg aaa ggt aaa gcg gct aag ttc gtt atc aac ctg aag aaa gtt      720
   Asn Leu Lys Gly Lys Ala Ala Lys Phe Val Ile Asn Leu Lys Lys Val
   225                 230                 235                 240
   gaa gaa cgc gaa ctg ccg gaa ctg acc gaa gaa ttc atc aaa cgt ttc      768
   Glu Glu Arg Glu Leu Pro Glu Leu Thr Glu Glu Phe Ile Lys Arg Phe
                   245                 250                 255
   ggc gtt gaa gat ggt tcc gta gcc ggt ctg cgc gct gaa gta cgt aaa      816
   Gly Val Glu Asp Gly Ser Val Ala Gly Leu Arg Ala Glu Val Arg Lys
               260                 265                 270
   aac atg gaa cgc gag ctg aaa ggc gcg gtg cgt aac cgc gtt aag tct      864
   Asn Met Glu Arg Glu Leu Lys Gly Ala Val Arg Asn Arg Val Lys Ser
           275                 280                 285
   cag gcg atc gaa ggt ctg gta aaa gct aac gac atc gac gta ccg tct      912
   Gln Ala Ile Glu Gly Leu Val Lys Ala Asn Asp Ile Asp Val Pro Ser
       290                 295                 300
   gcg ctg atc gac agc gaa atc gac gtt ctg cgt cgt cag gct gcg cag      960
   Ala Leu Ile Asp Ser Glu Ile Asp Val Leu Arg Arg Gln Ala Ala Gln
   305                 310                 315                 320
   cgt ttt ggt ggt aac gag aaa cag gct ctg gag ctg ccg cgc gaa ctg     1008
   Arg Phe Gly Gly Asn Glu Lys Gln Ala Leu Glu Leu Pro Arg Glu Leu
                   325                 330                 335
   ttc gaa gaa cag gct aag cgt cgt gta gtt gtc ggt ctg ctg ctc ggc      1056
   Phe Glu Glu Gln Ala Lys Arg Arg Val Val Val Gly Leu Leu Leu Gly
               340                 345                 350
   gaa gtc att cgt acc aac gag ctg aaa gcg gac gaa gaa cgc gtt aaa      1104
   Glu Val Ile Arg Thr Asn Glu Leu Lys Ala Asp Glu Glu Arg Val Lys
           355                 360                 365
   ggc ctg atc gaa gag atg gcc tct gct tac gaa gat ccg aaa gaa gtg      1152
   Gly Leu Ile Glu Glu Met Ala Ser Ala Tyr Glu Asp Pro Lys Glu Val
       370                 375                 380
   att gag ttc tac agc aaa aat aaa gag ctg atg gac aat atg cgt aac      1200
   Ile Glu Phe Tyr Ser Lys Asn Lys Glu Leu Met Asp Asn Met Arg Asn
   385                 390                 395                 400
   gtc gct ctg gaa gaa cag gct gtt gaa gcg gtt ctg gca aaa gcg aaa      1248
   Val Ala Leu Glu Glu Gln Ala Val Glu Ala Val Leu Ala Lys Ala Lys
                   405                 410                 415
   gtg tct gaa aaa gcc act tcc ttc aat gag ctg atg aac cag cag gcg      1296
   Val Ser Glu Lys Ala Thr Ser Phe Asn Glu Leu Met Asn Gln Gln Ala
               420                 425                 430
   taa                                                                  1299
<210> 4
   <211> 432
   <212> PRT
   <213> Salmonella enterica
<400> 4
   Met Gln Val Ser Val Glu Thr Thr Gln Gly Leu Gly Arg Arg Val Thr
   1               5                   10                  15
   Ile Thr Ile Ala Ala Asp Ser Ile Glu Thr Ala Val Lys Ser Glu Leu
               20                  25                  30
   Val Asn Val Ala Lys Lys Val Arg Ile Asp Gly Phe Arg Lys Gly Lys
           35                  40                  45
   Val Pro Met Asn Ile Val Ala Gln Arg Tyr Gly Ala Ser Val Arg Gln
       50                  55                  60
   Asp Val Leu Gly Asp Leu Met Ser Arg Asn Phe Val Asp Ala Ile Ile
   65                  70                  75                  80
   Lys Glu Lys Ile Asn Pro Ala Gly Ala Pro Asn Tyr Val Pro Gly Glu
                   85                  90                  95
   Tyr Lys Val Gly Glu Asp Phe Thr Tyr Ser Val Glu Phe Glu Val Tyr
               100                 105                 110
   Pro Glu Val Glu Leu Thr Gly Leu Glu Ser Ile Glu Val Glu Lys Pro
           115                 120                 125
   Val Val Glu Val Thr Asp Ala Asp Val Asp Val Met Leu Asp Thr Leu
       130                 135                 140
   Arg Lys Gln Gln Ala Thr Trp Lys Glu Lys Asp Gly Ala Ala Asp Ala
   145                 150                 155                 160
   Glu Asp Arg Val Thr Ile Asp Phe Thr Gly Ser Val Asp Gly Glu Glu
                   165                 170                 175
   Phe Glu Gly Gly Lys Ala Thr Asp Phe Val Leu Ala Met Gly Gln Gly
               180                 185                 190
   Arg Met Ile Pro Gly Phe Glu Asp Gly Val Lys Gly His Lys Ala Gly
           195                 200                 205
   Glu Glu Phe Thr Ile Asp Val Thr Phe Pro Glu Glu Tyr His Ala Glu
       210                 215                 220
   Asn Leu Lys Gly Lys Ala Ala Lys Phe Val Ile Asn Leu Lys Lys Val
   225                 230                 235                 240
   Glu Glu Arg Glu Leu Pro Glu Leu Thr Glu Glu Phe Ile Lys Arg Phe
                   245                 250                 255
   Gly Val Glu Asp Gly Ser Val Ala Gly Leu Arg Ala Glu Val Arg Lys
               260                 265                 270
   Asn Met Glu Arg Glu Leu Lys Gly Ala Val Arg Asn Arg Val Lys Ser
           275                 280                 285
   Gln Ala Ile Glu Gly Leu Val Lys Ala Asn Asp Ile Asp Val Pro Ser
       290                 295                 300
   Ala Leu Ile Asp Ser Glu Ile Asp Val Leu Arg Arg Gln Ala Ala Gln
   305                 310                 315                 320
   Arg Phe Gly Gly Asn Glu Lys Gln Ala Leu Glu Leu Pro Arg Glu Leu
                   325                 330                 335
   Phe Glu Glu Gln Ala Lys Arg Arg Val Val Val Gly Leu Leu Leu Gly
               340                 345                 350
   Glu Val Ile Arg Thr Asn Glu Leu Lys Ala Asp Glu Glu Arg Val Lys
           355                 360                 365
   Gly Leu Ile Glu Glu Met Ala Ser Ala Tyr Glu Asp Pro Lys Glu Val
       370                 375                 380
   Ile Glu Phe Tyr Ser Lys Asn Lys Glu Leu Met Asp Asn Met Arg Asn
   385                 390                 395                 400
   Val Ala Leu Glu Glu Gln Ala Val Glu Ala Val Leu Ala Lys Ala Lys
                   405                 410                 415
   Val Ser Glu Lys Ala Thr Ser Phe Asn Glu Leu Met Asn Gln Gln Ala
               420                 425                 430
<210> 5
   <211> 1305
   <212> DNA
   <213> Erwinia carotovora
<220>
   <221> CDS
   <222> (1)..(1305)
   <223> tig Kodierregion
<400> 5
   atg caa gtt tca gtt gaa acc act caa ggt ctg gga cgt cgc gta acg      48
   Met Gln Val Ser Val Glu Thr Thr Gln Gly Leu Gly Arg Arg Val Thr
   1               5                   10                  15
   att acc gtt gct gct gac atc att gag agt gcg gtg aag agc gag ctg      96
   Ile Thr Val Ala Ala Asp Ile Ile Glu Ser Ala Val Lys Ser Glu Leu
               20                  25                  30
   gtc aac gcg gcc aaa aaa gta cgt att gac ggt ttt cgt aaa ggc aaa     144
   Val Asn Ala Ala Lys Lys Val Arg Ile Asp Gly Phe Arg Lys Gly Lys
           35                  40                  45
   gtg ccg atg aat gtt gtt gct cag cgt tat ggt gcc tct gtg cgt cag     192
   Val Pro Met Asn Val Val Ala Gln Arg Tyr Gly Ala Ser Val Arg Gln
       50                  55                  60
   gac gta ttg ggt gac ctg atg cag cgc aac ttt gtt gac gct atc atc     240
   Asp Val Leu Gly Asp Leu Met Gln Arg Asn Phe Val Asp Ala Ile Ile
   65                  70                  75                  80
   aaa gaa aaa att aat ccg gtt ggc gcg cct aac tat acc ccc ggc gaa     288
   Lys Glu Lys Ile Asn Pro Val Gly Ala Pro Asn Tyr Thr Pro Gly Glu
                   85                  90                  95
   tat gcc gta ggt ggc gac ttc act tac act gta gag ttt gac gtg tac     336
   Tyr Ala Val Gly Gly Asp Phe Thr Tyr Thr Val Glu Phe Asp Val Tyr
               100                 105                 110
   ccg gaa gtc gaa ctg aaa ggt ctg gaa gca atc gaa gtt gaa aaa ccg     384
   Pro Glu Val Glu Leu Lys Gly Leu Glu Ala Ile Glu Val Glu Lys Pro
           115                 120                 125
   gtt gtt gaa gtg act gat gct gac gtt gat acc atg ctg gat acg ctg     432
   Val Val Glu Val Thr Asp Ala Asp Val Asp Thr Met Leu Asp Thr Leu
       130                 135                 140
   cgt aag caa cag gcg act tgg aaa gaa act gac cgt gct gcg aca gcc     480
   Arg Lys Gln Gln Ala Thr Trp Lys Glu Thr Asp Arg Ala Ala Thr Ala
   145                 150                 155                 160
   gaa gat cgt gca acg atc gat ttc act ggc tct atc gac ggt gaa gtc     528
   Glu Asp Arg Ala Thr Ile Asp Phe Thr Gly Ser Ile Asp Gly Glu Val
                   165                 170                 175
   ttc gaa ggc gga aaa gcg tcc gac ttc gtt ctg gca atg ggc cag agc     576
   Phe Glu Gly Gly Lys Ala Ser Asp Phe Val Leu Ala Met Gly Gln Ser
               180                 185                 190
   cgc atg atc cca ggt ttt gaa gat ggt atc gtt ggg cac aaa gcg ggt     624
   Arg Met Ile Pro Gly Phe Glu Asp Gly Ile Val Gly His Lys Ala Gly
           195                 200                 205
   gaa gaa ttc act atc aac gtg aat ttc cca gaa gac tac cac gct gaa      672
   Glu Glu Phe Thr Ile Asn Val Asn Phe Pro Glu Asp Tyr His Ala Glu
       210                 215                 220
   aat ctg aaa gga aaa gcg gct cag ttc gtt atc gtc ctg aag aaa gtt      720
   Asn Leu Lys Gly Lys Ala Ala Gln Phe Val Ile Val Leu Lys Lys Val
   225                 230                 235                 240
   gaa gag cgt gag ctg cct gaa ctg act gaa gaa ttc atc aaa cgt ttc      768
   Glu Glu Arg Glu Leu Pro Glu Leu Thr Glu Glu Phe Ile Lys Arg Phe
                   245                 250                 255
   ggc gtg gct gat ggt tct cag gaa ggt ctg cgt gct gaa gtc cgt aaa      816
   Gly Val Ala Asp Gly Ser Gln Glu Gly Leu Arg Ala Glu Val Arg Lys
               260                 265                 270
   aat atg gaa cgt gag ctg aaa ggc gcg gtg cgt aac cgt gtg aaa act      864
   Asn Met Glu Arg Glu Leu Lys Gly Ala Val Arg Asn Arg Val Lys Thr
           275                 280                 285
   cag gtt ctg gat gga ctg atc aac gcc aac gac atc gaa gtg cca gtt      912
   Gln Val Leu Asp Gly Leu Ile Asn Ala Asn Asp Ile Glu Val Pro Val
       290                 295                 300
   gca ctg atc gat ggc gaa atc gat gtt ctg cgc cgt cag gct gca cag      960
   Ala Leu Ile Asp Gly Glu Ile Asp Val Leu Arg Arg Gln Ala Ala Gln
   305                 310                 315                 320
   cgt ttt ggt ggc aac gag aag caa gcg ctg gaa tta ccg cgt gaa ctg     1008
   Arg Phe Gly Gly Asn Glu Lys Gln Ala Leu Glu Leu Pro Arg Glu Leu
                   325                 330                 335
   ttc gaa gag caa gct aaa cgc cgc gtt gtt atc ggt tta ttg ctg ggc     1056
   Phe Glu Glu Gln Ala Lys Arg Arg Val Val Ile Gly Leu Leu Leu Gly
               340                 345                 350
   gaa gtg atc agc agc aac gag ctg aaa gct gac gaa gca cgc gtt aat     1104
   Glu Val Ile Ser Ser Asn Glu Leu Lys Ala Asp Glu Ala Arg Val Asn
           355                 360                 365
   gta ctg atc gaa gag atg gct tct gct tac gaa gat ccg cag gaa gtt     1152
   Val Leu Ile Glu Glu Met Ala Ser Ala Tyr Glu Asp Pro Gln Glu Val
       370                 375                 380
   atc gag ttc tac agc aag aat aaa gag ctg ctg aac aac atg cgt aac     1200
   Ile Glu Phe Tyr Ser Lys Asn Lys Glu Leu Leu Asn Asn Met Arg Asn
   385                 390                 395                 400
   gtc gca ctg gaa gag caa gcg gtt gaa act gtg ctt gct aaa gcg aaa     1248
   Val Ala Leu Glu Glu Gln Ala Val Glu Thr Val Leu Ala Lys Ala Lys
                   405                 410                 415
   gtc gtt gag aaa tca gta agc ttc aac gaa ctg atg aat caa acg act     1296
   Val Val Glu Lys Ser Val Ser Phe Asn Glu Leu Met Asn Gln Thr Thr
               420                 425                 430
   acg gcg taa                                                         1305
   Thr Ala
<210> 6
   <211> 434
   <212> PRT
   <213> Erwinia carotovora
<400> 6
   Met Gln Val Ser Val Glu Thr Thr Gln Gly Leu Gly Arg Arg Val Thr
   1               5                   10                  15
   Ile Thr Val Ala Ala Asp Ile Ile Glu Ser Ala Val Lys Ser Glu Leu
               20                  25                  30
   Val Asn Ala Ala Lys Lys Val Arg Ile Asp Gly Phe Arg Lys Gly Lys
           35                  40                  45
   Val Pro Met Asn Val Val Ala Gln Arg Tyr Gly Ala Ser Val Arg Gln
       50                  55                  60
   Asp Val Leu Gly Asp Leu Met Gln Arg Asn Phe Val Asp Ala Ile Ile
   65                  70                  75                  80
   Lys Glu Lys Ile Asn Pro Val Gly Ala Pro Asn Tyr Thr Pro Gly Glu
                   85                  90                  95
   Tyr Ala Val Gly Gly Asp Phe Thr Tyr Thr Val Glu Phe Asp Val Tyr
               100                 105                 110
   Pro Glu Val Glu Leu Lys Gly Leu Glu Ala Ile Glu Val Glu Lys Pro
           115                 120                 125
   Val Val Glu Val Thr Asp Ala Asp Val Asp Thr Met Leu Asp Thr Leu
       130                 135                 140
   Arg Lys Gln Gln Ala Thr Trp Lys Glu Thr Asp Arg Ala Ala Thr Ala
   145                 150                 155                 160
   Glu Asp Arg Ala Thr Ile Asp Phe Thr Gly Ser Ile Asp Gly Glu Val
                   165                 170                 175
   Phe Glu Gly Gly Lys Ala Ser Asp Phe Val Leu Ala Met Gly Gln Ser
               180                 185                 190
   Arg Met Ile Pro Gly Phe Glu Asp Gly Ile Val Gly His Lys Ala Gly
           195                 200                 205
   Glu Glu Phe Thr Ile Asn Val Asn Phe Pro Glu Asp Tyr His Ala Glu
       210                 215                 220
   Asn Leu Lys Gly Lys Ala Ala Gln Phe Val Ile Val Leu Lys Lys Val
   225                 230                 235                 240
   Glu Glu Arg Glu Leu Pro Glu Leu Thr Glu Glu Phe Ile Lys Arg Phe
                   245                 250                 255
   Gly Val Ala Asp Gly Ser Gln Glu Gly Leu Arg Ala Glu Val Arg Lys
               260                 265                 270
   Asn Met Glu Arg Glu Leu Lys Gly Ala Val Arg Asn Arg Val Lys Thr
               275                 280                 285
   Gln Val Leu Asp Gly Leu Ile Asn Ala Asn Asp Ile Glu Val Pro Val
       290                 295                 300
   Ala Leu Ile Asp Gly Glu Ile Asp Val Leu Arg Arg Gln Ala Ala Gln
   305                 310                 315                 320
   Arg Phe Gly Gly Asn Glu Lys Gln Ala Leu Glu Leu Pro Arg Glu Leu
                   325                 330                 335
   Phe Glu Glu Gln Ala Lys Arg Arg Val Val Ile Gly Leu Leu Leu Gly
               340                 345                 350
   Glu Val Ile Ser Ser Asn Glu Leu Lys Ala Asp Glu Ala Arg Val Asn
           355                 360                 365
   Val Leu Ile Glu Glu Met Ala Ser Ala Tyr Glu Asp Pro Gln Glu Val
       370                 375                 380
   Ile Glu Phe Tyr Ser Lys Asn Lys Glu Leu Leu Asn Asn Met Arg Asn
   385                 390                 395                 400
   Val Ala Leu Glu Glu Gln Ala Val Glu Thr Val Leu Ala Lys Ala Lys
                   405                 410                 415
   Val Val Glu Lys Ser Val Ser Phe Asn Glu Leu Met Asn Gln Thr Thr
               420                 425                 430
   Thr Ala
<210> 7
   <211> 2463
   <212> DNA
   <213> Escherichia coli
<220>
   <221> CDS
   <222> (1)..(2463)
   <223> thrA-Kodierregion
<400> 7
   atg cga gtg ttg aag ttc ggc ggt aca tca gtg gca aat gca gaa cgt        48
   Met Arg Val Leu Lys Phe Gly Gly Thr Ser Val Ala Asn Ala Glu Arg
   1               5                   10                  15
   ttt ctg cgt gtt gcc gat att ctg gaa agc aat gcc agg cag ggg cag        96
   Phe Leu Arg Val Ala Asp Ile Leu Glu Ser Asn Ala Arg Gln Gly Gln
               20                  25                  30
   gtg gcc acc gtc ctc tct gcc ccc gcc aaa atc acc aac cac ctg gtg       144
   Val Ala Thr Val Leu Ser Ala Pro Ala Lys Ile Thr Asn His Leu Val
           35                  40                  45
   gcg atg att gaa aaa acc att agc ggc cag gat gct tta ccc aat atc       192
   Ala Met Ile Glu Lys Thr Ile Ser Gly Gln Asp Ala Leu Pro Asn Ile
       50                  55                  60
   agc gat gcc gaa cgt att ttt gcc gaa ctt ttg acg gga ctc gcc gcc      240
   Ser Asp Ala Glu Arg Ile Phe Ala Glu Leu Leu Thr Gly Leu Ala Ala
   65                  70                  75                  80
   gcc cag ccg ggg ttc ccg ctg gcg caa ttg aaa act ttc gtc gat cag      288
   Ala Gln Pro Gly Phe Pro Leu Ala Gln Leu Lys Thr Phe Val Asp Gln
                   85                  90                  95
   gaa ttt gcc caa ata aaa cat gtc ctg cat ggc att agt ttg ttg ggg      336
   Glu Phe Ala Gln Ile Lys His Val Leu His Gly Ile Ser Leu Leu Gly
               100                 105                 110
   cag tgc ccg gat agc atc aac gct gcg ctg att tgc cgt ggc gag aaa      384
   Gln Cys Pro Asp Ser Ile Asn Ala Ala Leu Ile Cys Arg Gly Glu Lys
           115                 120                 125
   atg tcg atc gcc att atg gcc ggc gta tta gaa gcg cgc ggt cac aac      432
   Met Ser Ile Ala Ile Met Ala Gly Val Leu Glu Ala Arg Gly His Asn
       130                 135                 140
   gtt act gtt atc gat ccg gtc gaa aaa ctg ctg gca gtg ggg cat tac      480
   Val Thr Val Ile Asp Pro Val Glu Lys Leu Leu Ala Val Gly His Tyr
   145                 150                 155                 160
   ctc gaa tct acc gtc gat att gct gag tcc acc cgc cgt att gcg gca      528
   Leu Glu Ser Thr Val Asp Ile Ala Glu Ser Thr Arg Arg Ile Ala Ala
                   165                 170                 175
   agc cgc att ccg gct gat cac atg gtg ctg atg gca ggt ttc acc gcc      576
   Ser Arg Ile Pro Ala Asp His Met Val Leu Met Ala Gly Phe Thr Ala
               180                 185                 190
   ggt aat gaa aaa ggc gaa ctg gtg gtg ctt gga cgc aac ggt tcc gac      624
   Gly Asn Glu Lys Gly Glu Leu Val Val Leu Gly Arg Asn Gly Ser Asp
           195                 200                 205
   tac tct gct gcg gtg ctg gct gcc tgt tta cgc gcc gat tgt tgc gag      672
   Tyr Ser Ala Ala Val Leu Ala Ala Cys Leu Arg Ala Asp Cys Cys Glu
       210                 215                 220
   att tgg acg gac gtt gac ggg gtc tat acc tgc gac ccg cgt cag gtg      720
   Ile Trp Thr Asp Val Asp Gly Val Tyr Thr Cys Asp Pro Arg Gln Val
   225                 230                 235                 240
   ccc gat gcg agg ttg ttg aag tcg atg tcc tac cag gaa gcg atg gag      768
   Pro Asp Ala Arg Leu Leu Lys Ser Met Ser Tyr Gln Glu Ala Met Glu
                   245                 250                 255
   ctt tcc tac ttc ggc gct aaa gtt ctt cac ccc cgc acc att acc ccc      816
   Leu Ser Tyr Phe Gly Ala Lys Val Leu His Pro Arg Thr Ile Thr Pro
               260                 265                 270
   atc gcc cag ttc cag atc cct tgc ctg att aaa aat acc gga aat cct      864
   Ile Ala Gln Phe Gln Ile Pro Cys Leu Ile Lys Asn Thr Gly Asn Pro
           275                 280                 285
   caa gca cca ggt acg ctc att ggt gcc agc cgt gat gaa gac gaa tta      912
   Gln Ala Pro Gly Thr Leu Ile Gly Ala Ser Arg Asp Glu Asp Glu Leu
       290                 295                 300
   ccg gtc aag ggc att tcc aat ctg aat aac atg gca atg ttc agc gtt      960
   Pro Val Lys Gly Ile Ser Asn Leu Asn Asn Met Ala Met Phe Ser Val
   305                 310                 315                 320
   tct ggt ccg ggg atg aaa ggg atg gtc ggc atg gcg gcg cgc gtc ttt     1008
   Ser Gly Pro Gly Met Lys Gly Met Val Gly Met Ala Ala Arg Val Phe
                   325                 330                 335
   gca gcg atg tca cgc gcc cgt att tcc gtg gtg ctg att acg caa tca     1056
   Ala Ala Met Ser Arg Ala Arg Ile Ser Val Val Leu Ile Thr Gln Ser
               340                 345                 350
   tct tcc gaa tac agc atc agt ttc tgc gtt cca caa agc gac tgt gtg      1104
   Ser Ser Glu Tyr Ser Ile Ser Phe Cys Val Pro Gln Ser Asp Cys Val
           355                 360                 365
   cga gct gaa cgg gca atg cag gaa gag ttc tac ctg gaa ctg aaa gaa      1152
   Arg Ala Glu Arg Ala Met Gln Glu Glu Phe Tyr Leu Glu Leu Lys Glu
       370                 375                 380
   ggc tta ctg gag ccg ctg gca gtg acg gaa cgg ctg gcc att atc tcg      1200
   Gly Leu Leu Glu Pro Leu Ala Val Thr Glu Arg Leu Ala Ile Ile Ser
   385                 390                 395                 400
   gtg gta ggt gat ggt atg cgc acc ttg cgt ggg atc tcg gcg aaa ttc      1248
   Val Val Gly Asp Gly Met Arg Thr Leu Arg Gly Ile Ser Ala Lys Phe
                   405                 410                 415
   ttt gcc gca ctg gcc cgc gcc aat atc aac att gtc gcc att gct cag      1296
   Phe Ala Ala Leu Ala Arg Ala Asn Ile Asn Ile Val Ala Ile Ala Gln
               420                 425                 430
   gga tct tct gaa cgc tca atc tct gtc gtg gta aat aac gat gat gcg      1344
   Gly Ser Ser Glu Arg Ser Ile Ser Val Val Val Asn Asn Asp Asp Ala
           435                 440                 445
   acc act ggc gtg cgc gtt act cat cag atg ctg ttc aat acc gat cag      1392
   Thr Thr Gly Val Arg Val Thr His Gln Met Leu Phe Asn Thr Asp Gln
       450                 455                 460
   gtt atc gaa gtg ttt gtg att ggc gtc ggt ggc gtt ggc ggt gcg ctg      1440
   Val Ile Glu Val Phe Val Ile Gly Val Gly Gly Val Gly Gly Ala Leu
   465                 470                 475                 480
   ctg gag caa ctg aag cgt cag caa agc tgg ctg aag aat aaa cat atc      1488
   Leu Glu Gln Leu Lys Arg Gln Gln Ser Trp Leu Lys Asn Lys His Ile
                   485                 490                 495
   gac tta cgt gtc tgc ggt gtt gcc aac tcg aag gct ctg ctc acc aat      1536
   Asp Leu Arg Val Cys Gly Val Ala Asn Ser Lys Ala Leu Leu Thr Asn
               500                 505                 510
   gta cat ggc ctt aat ctg gaa aac tgg cag gaa gaa ctg gcg caa gcc      1584
   Val His Gly Leu Asn Leu Glu Asn Trp Gln Glu Glu Leu Ala Gln Ala
           515                 520                 525
   aaa gag ccg ttt aat ctc ggg cgc tta att cgc ctc gtg aaa gaa tat      1632
   Lys Glu Pro Phe Asn Leu Gly Arg Leu Ile Arg Leu Val Lys Glu Tyr
       530                 535                 540
   cat ctg ctg aac ccg gtc att gtt gac tgc act tcc agc cag gca gtg      1680
   His Leu Leu Asn Pro Val Ile Val Asp Cys Thr Ser Ser Gln Ala Val
   545                 550                 555                 560
   gcg gat caa tat gcc gac ttc ctg cgc gaa ggt ttc cac gtt gtc acg      1728
   Ala Asp Gln Tyr Ala Asp Phe Leu Arg Glu Gly Phe His Val Val Thr
                   565                 570                 575
   ccg aac aaa aag gcc aac acc tcg tcg atg gat tac tac cat cag ttg      1776
   Pro Asn Lys Lys Ala Asn Thr Ser Ser Met Asp Tyr Tyr His Gln Leu
               580                 585                 590
   cgt tat gcg gcg gaa aaa tcg cgg cgt aaa ttc ctc tat gac acc aac      1824
   Arg Tyr Ala Ala Glu Lys Ser Arg Arg Lys Phe Leu Tyr Asp Thr Asn
           595                 600                 605
   gtt ggg gct gga tta ccg gtt att gag aac ctg caa aat ctg ctc aat      1872
   Val Gly Ala Gly Leu Pro Val Ile Glu Asn Leu Gln Asn Leu Leu Asn
       610                 615                 620
   gca ggt gat gaa ttg atg aag ttc tcc ggc att ctt tct ggt tcg ctt      1920
   Ala Gly Asp Glu Leu Met Lys Phe Ser Gly Ile Leu Ser Gly Ser Leu
   625                 630                 635                 640
   tct tat atc ttc ggc aag tta gac gaa ggc atg agt ttc tcc gag gcg      1968
   Ser Tyr Ile Phe Gly Lys Leu Asp Glu Gly Met Ser Phe Ser Glu Ala
                   645                 650                 655
   acc acg ctg gcg cgg gaa atg ggt tat acc gaa ccg gac ccg cga gat      2016
   Thr Thr Leu Ala Arg Glu Met Gly Tyr Thr Glu Pro Asp Pro Arg Asp
               660                 665                 670
   gat ctt tct ggt atg gat gtg gcg cgt aaa cta ttg att ctc gct cgt      2064
   Asp Leu Ser Gly Met Asp Val Ala Arg Lys Leu Leu Ile Leu Ala Arg
           675                 680                 685
   gaa acg gga cgt gaa ctg gag ctg gcg gat att gaa att gaa cct gtg      2112
   Glu Thr Gly Arg Glu Leu Glu Leu Ala Asp Ile Glu Ile Glu Pro Val
       690                 695                 700
   ctg ccc gca gag ttt aac gcc gag ggt gat gtt gcc gct ttt atg gcg      2160
   Leu Pro Ala Glu Phe Asn Ala Glu Gly Asp Val Ala Ala Phe Met Ala
   705                 710                 715                 720
   aat ctg tca caa ctc gac gat ctc ttt gcc gcg cgc gtg gcg aag gcc      2208
   Asn Leu Ser Gln Leu Asp Asp Leu Phe Ala Ala Arg Val Ala Lys Ala
                   725                 730                 735
   cgt gat gaa gga aaa gtt ttg cgc tat gtt ggc aat att gat gaa gat      2256
   Arg Asp Glu Gly Lys Val Leu Arg Tyr Val Gly Asn Ile Asp Glu Asp
               740                 745                 750
   ggc gtc tgc cgc gtg aag att gcc gaa gtg gat ggt aat gat ccg ctg      2304
   Gly Val Cys Arg Val Lys Ile Ala Glu Val Asp Gly Asn Asp Pro Leu
           755                 760                 765
   ttc aaa gtg aaa aat ggc gaa aac gcc ctg gcc ttc tat agc cac tat      2352
   Phe Lys Val Lys Asn Gly Glu Asn Ala Leu Ala Phe Tyr Ser His Tyr
       770                 775                 780
   tat cag ccg ctg ccg ttg gta ctg cgc gga tat ggt gcg ggc aat gac      2400
   Tyr Gln Pro Leu Pro Leu Val Leu Arg Gly Tyr Gly Ala Gly Asn Asp
   785                 790                 795                 800
   gtt aca gct gcc ggt gtc ttt gct gat ctg cta cgt acc ctc tca tgg      2448
   Val Thr Ala Ala Gly Val Phe Ala Asp Leu Leu Arg Thr Leu Ser Trp
                   805                 810                 815
   aag tta gga gtc tga                                                  2463
   Lys Leu Gly Val
               820
<210> 8
   <211> 820
   <212> PRT
   <213> Escherichia coli
<400> 8
   Met Arg Val Leu Lys Phe Gly Gly Thr Ser Val Ala Asn Ala Glu Arg
   1               5                   10                  15
   Phe Leu Arg Val Ala Asp Ile Leu Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Glu Lys Thr Ile Ser Gly Gln Asp Ala Leu Pro Asn Ile
       50                  55                  60
   Ser Asp Ala Glu Arg Ile Phe Ala Glu Leu Leu Thr Gly Leu Ala Ala
   65                  70                  75                  80
   Ala Gln Pro Gly Phe Pro Leu Ala Gln Leu Lys Thr Phe Val Asp Gln
                   85                  90                  95
   Glu Phe Ala Gln Ile Lys His Val Leu His Gly Ile Ser Leu Leu Gly
               100                 105                 110
   Gln Cys Pro Asp Ser Ile Asn Ala Ala Leu Ile Cys Arg Gly Glu Lys
           115                 120                 125
   Met Ser Ile Ala Ile Met Ala Gly Val Leu Glu Ala Arg Gly His Asn
       130                 135                 140
   Val Thr Val Ile Asp Pro Val Glu Lys Leu Leu Ala Val Gly His Tyr
   145                 150                 155                 160
   Leu Glu Ser Thr Val Asp Ile Ala Glu Ser Thr Arg Arg Ile Ala Ala
                   165                 170                 175
   Ser Arg Ile Pro Ala Asp His Met Val Leu Met Ala Gly Phe Thr Ala
               180                 185                 190
   Gly Asn Glu Lys Gly Glu Leu Val Val Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Thr Cys Asp Pro Arg Gln Val
   225                 230                 235                 240
   Pro Asp Ala Arg Leu Leu Lys Ser Met Ser Tyr Gln Glu Ala Met Glu
                   245                 250                 255
   Leu Ser Tyr Phe Gly Ala Lys Val Leu His Pro Arg Thr Ile Thr Pro
               260                 265                 270
   Ile Ala Gln Phe Gln Ile Pro Cys Leu Ile Lys Asn Thr Gly Asn Pro
           275                 280                 285
   Gln Ala Pro Gly Thr Leu Ile Gly Ala Ser Arg Asp Glu Asp Glu Leu
       290                 295                 300
   Pro Val Lys Gly Ile Ser Asn Leu Asn Asn Met Ala Met Phe Ser Val
   305                 310                 315                 320
   Ser Gly Pro Gly Met Lys Gly Met Val Gly Met Ala Ala Arg Val Phe
                   325                 330                 335
   Ala Ala Met Ser Arg Ala Arg Ile Ser Val Val Leu Ile Thr Gln Ser
               340                 345                 350
   Ser Ser Glu Tyr Ser Ile Ser Phe Cys Val Pro Gln Ser Asp Cys Val
           355                 360                 365
   Arg Ala Glu Arg Ala Met Gln Glu Glu Phe Tyr Leu Glu Leu Lys Glu
       370                 375                 380
   Gly Leu Leu Glu Pro Leu Ala Val Thr Glu Arg Leu Ala Ile Ile Ser
   385                 390                 395                 400
   Val Val Gly Asp Gly Met Arg Thr Leu Arg Gly Ile Ser Ala Lys Phe
                   405                 410                 415
   Phe Ala Ala Leu Ala Arg Ala Asn Ile Asn Ile Val Ala Ile Ala Gln
               420                 425                 430
   Gly Ser Ser Glu Arg Ser Ile Ser Val Val Val Asn Asn Asp Asp Ala
           435                 440                 445
   Thr Thr Gly Val Arg Val Thr His Gln Met Leu Phe Asn Thr Asp Gln
       450                 455                 460
   Val Ile Glu Val Phe Val Ile Gly Val Gly Gly Val Gly Gly Ala Leu
   465                 470                 475                 480
   Leu Glu Gln Leu Lys Arg Gln Gln Ser Trp Leu Lys Asn Lys His Ile
                   485                 490                 495
   Asp Leu Arg Val Cys Gly Val Ala Asn Ser Lys Ala Leu Leu Thr Asn
               500                 505                 510
   Val His Gly Leu Asn Leu Glu Asn Trp Gln Glu Glu Leu Ala Gln Ala
           515                 520                 525
   Lys Glu Pro Phe Asn Leu Gly Arg Leu Ile Arg Leu Val Lys Glu Tyr
       530                 535                 540
   His Leu Leu Asn Pro Val Ile Val Asp Cys Thr Ser Ser Gln Ala Val
   545                 550                 555                 560
   Ala Asp Gln Tyr Ala Asp Phe Leu Arg Glu Gly Phe His Val Val Thr
                   565                 570                 575
   Pro Asn Lys Lys Ala Asn Thr Ser Ser Met Asp Tyr Tyr His Gln Leu
               580                 585                 590
   Arg Tyr Ala Ala Glu Lys Ser Arg Arg Lys Phe Leu Tyr Asp Thr Asn
           595                 600                 605
   Val Gly Ala Gly Leu Pro Val Ile Glu Asn Leu Gln Asn Leu Leu Asn
       610                 615                 620
   Ala Gly Asp Glu Leu Met Lys Phe Ser Gly Ile Leu Ser Gly Ser Leu
   625                 630                 635                 640
   Ser Tyr Ile Phe Gly Lys Leu Asp Glu Gly Met Ser Phe Ser Glu Ala
                   645                 650                 655
   Thr Thr Leu Ala Arg Glu Met Gly Tyr Thr Glu Pro Asp Pro Arg Asp
               660                 665                 670
   Asp Leu Ser Gly Met Asp Val Ala Arg Lys Leu Leu Ile Leu Ala Arg
           675                 680                 685
   Glu Thr Gly Arg Glu Leu Glu Leu Ala Asp Ile Glu Ile Glu Pro Val
       690                 695                 700
   Leu Pro Ala Glu Phe Asn Ala Glu Gly Asp Val Ala Ala Phe Met Ala
   705                 710                 715                 720
   Asn Leu Ser Gln Leu Asp Asp Leu Phe Ala Ala Arg Val Ala Lys Ala
                   725                 730                 735
   Arg Asp Glu Gly Lys Val Leu Arg Tyr Val Gly Asn Ile Asp Glu Asp
               740                 745                 750
   Gly Val Cys Arg Val Lys Ile Ala Glu Val Asp Gly Asn Asp Pro Leu
           755                 760                 765
   Phe Lys Val Lys Asn Gly Glu Asn Ala Leu Ala Phe Tyr Ser His Tyr
       770                 775                 780
   Tyr Gln Pro Leu Pro Leu Val Leu Arg Gly Tyr Gly Ala Gly Asn Asp
   785                 790                 795                 800
   Val Thr Ala Ala Gly Val Phe Ala Asp Leu Leu Arg Thr Leu Ser Trp
                   805                 810                 815
   Lys Leu Gly Val
               820
<210> 9
   <211> 2460
   <212> DNA
   <213> Serratia marcescens
<220>
   <221> CDS
   <222> (1)..(2460)
   <223> thrA-Kodierregion
<400> 9
   atg cga gtg ctg aaa ttt ggc gga acc tcg gta gcg aat gcg gaa cgt       48
   Met Arg Val Leu Lys Phe Gly Gly Thr Ser Val Ala Asn Ala Glu Arg
   1               5                   10                  15
   ttt ctg cgc gtc gcc gac atc atg gag agt aac gcg cgt cag gga cag       96
   Phe Leu Arg Val Ala Asp Ile Met Glu Ser Asn Ala Arg Gln Gly Gln
               20                  25                  30
   gta gcc acg gtc ttg tcc gcc ccc gca aag atc acc aac cat ctg gtg      144
   Val Ala Thr Val Leu Ser Ala Pro Ala Lys Ile Thr Asn His Leu Val
           35                  40                  45
   gcg atg atc gac aaa acg gtg gcg ggc cag gac att ctg ccg aat atg      192
   Ala Met Ile Asp Lys Thr Val Ala Gly Gln Asp Ile Leu Pro Asn Met
       50                  55                  60
   agc gac gcc gag cgg atc ttt gcc gac ctg ctg agc gga ctg gcg cag      240
   Ser Asp Ala Glu Arg Ile Phe Ala Asp Leu Leu Ser Gly Leu Ala Gln
   65                  70                  75                  80
   gcg ctg ccg ggc ttt gaa tac gat cgt ttg aaa ggc gtg gtc gat cag      288
   Ala Leu Pro Gly Phe Glu Tyr Asp Arg Leu Lys Gly Val Val Asp Gln
                   85                  90                  95
   gaa ttc gcg cag ctc aaa cag gtg ctg cat ggc gtc tcg ctg ctg ggg      336
   Glu Phe Ala Gln Leu Lys Gln Val Leu His Gly Val Ser Leu Leu Gly
               100                 105                 110
   cag tgc ccg gac agc gtg aac gcg gcg atc atc tgc cgt ggc gaa aag      384
   Gln Cys Pro Asp Ser Val Asn Ala Ala Ile Ile Cys Arg Gly Glu Lys
           115                 120                 125
   ctc tcc atc gcc atc atg gaa ggg gtg ttc cgc gcc aag ggt tat ccg      432
   Leu Ser Ile Ala Ile Met Glu Gly Val Phe Arg Ala Lys Gly Tyr Pro
       130                 135                 140
   gtc acg gtg atc aac ccg gtg gag aaa ctg ctg gcg cag ggc cac tac      480
   Val Thr Val Ile Asn Pro Val Glu Lys Leu Leu Ala Gln Gly His Tyr
   145                 150                 155                 160
   ctg gag tcc acc gtg gac atc gcc gag tct acg ctg cgc atc gcc gcc      528
   Leu Glu Ser Thr Val Asp Ile Ala Glu Ser Thr Leu Arg Ile Ala Ala
                   165                 170                 175
   gcg gcg atc ccg gcc gat cac atc gtg ctg atg gcc ggt ttc acc gcc      576
   Ala Ala Ile Pro Ala Asp His Ile Val Leu Met Ala Gly Phe Thr Ala
               180                 185                 190
   ggt aat gat aag ggc gag ctg gtg gtg ctg ggc cgc aac ggc tcc gac      624
   Gly Asn Asp Lys Gly Glu Leu Val Val Leu Gly Arg Asn Gly Ser Asp
           195                 200                 205
   tat tcc gcc gcg gtg ctg gcc gcc tgc ctg cgc gcc gac tgt tgc gag      672
   Tyr Ser Ala Ala Val Leu Ala Ala Cys Leu Arg Ala Asp Cys Cys Glu
       210                 215                 220
   atc tgg acc gac gtc gac ggc gtt tat acc tgc gat ccg cgc acc gtg      720
   Ile Trp Thr Asp Val Asp Gly Val Tyr Thr Cys Asp Pro Arg Thr Val
   225                 230                 235                 240
   ccg gac gcc agg tta ctg aaa tcg atg tcg tac cag gaa gcg atg gag      768
   Pro Asp Ala Arg Leu Leu Lys Ser Met Ser Tyr Gln Glu Ala Met Glu
                   245                 250                 255
   ctt tcc tat ttc ggc gcc aaa gtg tta cac ccc cgc acc atc act ccg      816
   Leu Ser Tyr Phe Gly Ala Lys Val Leu His Pro Arg Thr Ile Thr Pro
               260                 265                 270
   att gcc cag ttt caa atc cct tgc ctg att aaa aac acc tcc aac ccg      864
   Ile Ala Gln Phe Gln Ile Pro Cys Leu Ile Lys Asn Thr Ser Asn Pro
           275                 280                 285
   cag gcc ccc ggc acg ctg atc ggc aaa gac agc acc gat gcc gat atg      912
   Gln Ala Pro Gly Thr Leu Ile Gly Lys Asp Ser Thr Asp Ala Asp Met
       290                 295                 300
   ccg gtg aag ggc atc acc aac ctg aac aac atg gcg atg atc aac gtc      960
   Pro Val Lys Gly Ile Thr Asn Leu Asn Asn Met Ala Met Ile Asn Val
   305                 310                 315                 320
   tcc ggc ccg ggc atg aaa ggc atg gtc ggc atg gcc gca cgg gtc ttc     1008
   Ser Gly Pro Gly Met Lys Gly Met Val Gly Met Ala Ala Arg Val Phe
                   325                 330                 335
   gcc gtg atg tcg cgc gcc ggc att tcg gtg gtg ctg atc acc caa tcc     1056
   Ala Val Met Ser Arg Ala Gly Ile Ser Val Val Leu Ile Thr Gln Ser
               340                 345                 350
   tct tcc gaa tac agc atc agc ttc tgc gtg ccg cag ggt gaa ctg cag     1104
   Ser Ser Glu Tyr Ser Ile Ser Phe Cys Val Pro Gln Gly Glu Leu Gln
           355                 360                 365
   cgc gcc cgt cgc gcg ctg gaa gag gag ttc tat ctg gag ctg aaa gac     1152
   Arg Ala Arg Arg Ala Leu Glu Glu Glu Phe Tyr Leu Glu Leu Lys Asp
       370                 375                 380
   ggc gtg ctg gat ccg ctg gac gtg atg gaa cgc ctg gcg atc atc tcg     1200
   Gly Val Leu Asp Pro Leu Asp Val Met Glu Arg Leu Ala Ile Ile Ser
   385                 390                 395                 400
   gtg gtt ggc gac ggc atg cgc acc ctg cgc ggc att tcc gca cgc ttc     1248
   Val Val Gly Asp Gly Met Arg Thr Leu Arg Gly Ile Ser Ala Arg Phe
                   405                 410                 415
   ttc tcc gcg ctg gcg cgc gcc aac atc aat atc gtt gcc atc gcc caa     1296
   Phe Ser Ala Leu Ala Arg Ala Asn Ile Asn Ile Val Ala Ile Ala Gln
               420                 425                 430
   ggg tct tcc gaa cgt tcc att tcg gtg gtg gtc agc aat gat tcc gcc     1344
   Gly Ser Ser Glu Arg Ser Ile Ser Val Val Val Ser Asn Asp Ser Ala
           435                 440                 445
   acc acc ggc gtg cgc gtc agc cac cag atg ctg ttc aac acc gat cag     1392
   Thr Thr Gly Val Arg Val Ser His Gln Met Leu Phe Asn Thr Asp Gln
       450                 455                 460
   gtg atc gaa gtg ttc gtc atc ggc gtc ggc ggc gtc ggc ggg gcg ctg     1440
   Val Ile Glu Val Phe Val Ile Gly Val Gly Gly Val Gly Gly Ala Leu
   465                 470                 475                 480
   atc gag cag atc tat cgc cag cag ccg tgg ctg aag caa aaa cat atc     1488
   Ile Glu Gln Ile Tyr Arg Gln Gln Pro Trp Leu Lys Gln Lys His Ile
                   485                 490                 495
   gat ctg cgg gtg tgc ggc atc gcc aac tcg cgc gtg atg ctg acc aat     1536
   Asp Leu Arg Val Cys Gly Ile Ala Asn Ser Arg Val Met Leu Thr Asn
               500                 505                 510
   gtg cac ggc atc gcg ctg gac agc tgg cgc gac gcg ctg gcc ggc gcg     1584
   Val His Gly Ile Ala Leu Asp Ser Trp Arg Asp Ala Leu Ala Gly Ala
           515                 520                 525
   cag gag ccg ttc aat ctc ggc cgt ctg atc cgg ctg gtg aag gaa tat      1632
   Gln Glu Pro Phe Asn Leu Gly Arg Leu Ile Arg Leu Val Lys Glu Tyr
       530                 535                 540
   cac ctg ctg aac ccg gtg atc gtc gac tgt acc tcc agt cag gcg gtg      1680
   His Leu Leu Asn Pro Val Ile Val Asp Cys Thr Ser Ser Gln Ala Val
   545                 550                 555                 560
   gcc gat cag tac gtg gac ttc ctg gcg gac ggc ttc cat gtg gtg acg      1728
   Ala Asp Gln Tyr Val Asp Phe Leu Ala Asp Gly Phe His Val Val Thr
                   565                 570                 575
   ccg aac aaa aag gcc aac acc tcg tcg atg aac tat tat cag caa ctg      1776
   Pro Asn Lys Lys Ala Asn Thr Ser Ser Met Asn Tyr Tyr Gln Gln Leu
               580                 585                 590
   cgc gcg gcc gcc gcc ggt tcg cat cgc aag ttc ctg tac gac acc aac      1824
   Arg Ala Ala Ala Ala Gly Ser His Arg Lys Phe Leu Tyr Asp Thr Asn
           595                 600                 605
   gtc ggt gcc ggt ctg ccg gtg att gag aac ctg caa aac ctg ctg aac      1872
   Val Gly Ala Gly Leu Pro Val Ile Glu Asn Leu Gln Asn Leu Leu Asn
       610                 615                 620
   gcc ggt gat gaa ctg gta cgt ttc tcc ggt atc ctg tcc ggc tcg ctg      1920
   Ala Gly Asp Glu Leu Val Arg Phe Ser Gly Ile Leu Ser Gly Ser Leu
   625                 630                 635                 640
   tcc ttt atc ttc ggc aag ctg gac gaa ggg ctg tcg ctg tcg gcg gcg      1968
   Ser Phe Ile Phe Gly Lys Leu Asp Glu Gly Leu Ser Leu Ser Ala Ala
                   645                 650                 655
   acc ctg cag gcc cgg gcc aac ggc tac acc gag ccg gat ccg cgc gac      2016
   Thr Leu Gln Ala Arg Ala Asn Gly Tyr Thr Glu Pro Asp Pro Arg Asp
               660                 665                 670
   gat ctg tcc ggg atg gat gtg gcg cgt aag ctg ctg atc ctg gcg cgc      2064
   Asp Leu Ser Gly Met Asp Val Ala Arg Lys Leu Leu Ile Leu Ala Arg
           675                 680                 685
   gag gcc ggt tac aaa ctg gaa ctg agc gat atc gag gtc gag ccg gtg      2112
   Glu Ala Gly Tyr Lys Leu Glu Leu Ser Asp Ile Glu Val Glu Pro Val
       690                 695                 700
   ctg ccg ccg tcc ttc gac gcg tcg ggc gac gtg gac acc ttc ctg gcg      2160
   Leu Pro Pro Ser Phe Asp Ala Ser Gly Asp Val Asp Thr Phe Leu Ala
   705                 710                 715                 720
   cgg ttg ccg gag ctg gat aaa gag ttc gcg cgc aac gtg gct aac gcc      2208
   Arg Leu Pro Glu Leu Asp Lys Glu Phe Ala Arg Asn Val Ala Asn Ala
                   725                 730                 735
   gcc gag caa ggc aag gtg ctg cgc tat gtc ggc ctg atc gac gaa ggg      2256
   Ala Glu Gln Gly Lys Val Leu Arg Tyr Val Gly Leu Ile Asp Glu Gly
               740                 745                 750
   cgc tgc aag gtg cgc att gag gcg gtg gac ggc aac gat ccg ttg tat      2304
   Arg Cys Lys Val Arg Ile Glu Ala Val Asp Gly Asn Asp Pro Leu Tyr
           755                 760                 765
   aaa gtg aag aac ggc gag aac gcg ctg gcc ttc tac agc cgc tac tat      2352
   Lys Val Lys Asn Gly Glu Asn Ala Leu Ala Phe Tyr Ser Arg Tyr Tyr
       770                 775                 780
   cag ccg ttg ccg ctg gtg ctg cgc ggc tac ggc gcc ggt aac gat gtg      2400
   Gln Pro Leu Pro Leu Val Leu Arg Gly Tyr Gly Ala Gly Asn Asp Val
   785                 790                 795                 800
   act gca gcg ggc gta ttc gcc gat ctg ctg cgc aca ctg tca tgg aag      2448
   Thr Ala Ala Gly Val Phe Ala Asp Leu Leu Arg Thr Leu Ser Trp Lys
                   805                 810                 815
   ttg gga gtt taa                                                  2460
   Leu Gly Val
<210> 10
   <211> 819
   <212> PRT
   <213> Serratia marcescens
<400> 10
   Met Arg Val Leu Lys Phe Gly Gly Thr Ser Val Ala Asn Ala Glu Arg
   1               5                   10                  15
   Phe Leu Arg Val Ala Asp Ile Met Glu Ser Asn Ala Arg Gln Gly Gln
               20                  25                  30
   Val Ala Thr Val Leu Ser Ala Pro Ala Lys Ile Thr Asn His Leu Val
           35                  40                  45
   Ala Met Ile Asp Lys Thr Val Ala Gly Gln Asp Ile Leu Pro Asn Met
       50                  55                  60
   Ser Asp Ala Glu Arg Ile Phe Ala Asp Leu Leu Ser Gly Leu Ala Gln
   65                  70                  75                  80
   Ala Leu Pro Gly Phe Glu Tyr Asp Arg Leu Lys Gly Val Val Asp Gln
                   85                  90                  95
   Glu Phe Ala Gln Leu Lys Gln Val Leu His Gly Val Ser Leu Leu Gly
               100                 105                 110
   Gln Cys Pro Asp Ser Val Asn Ala Ala Ile Ile Cys Arg Gly Glu Lys
           115                 120                 125
   Leu Ser Ile Ala Ile Met Glu Gly Val Phe Arg Ala Lys Gly Tyr Pro
       130                 135                 140
   Val Thr Val Ile Asn Pro Val Glu Lys Leu Leu Ala Gln Gly His Tyr
   145                 150                 155                 160
   Leu Glu Ser Thr Val Asp Ile Ala Glu Ser Thr Leu Arg Ile Ala Ala
                   165                 170                 175
   Ala Ala Ile Pro Ala Asp His Ile Val Leu Met Ala Gly Phe Thr Ala
               180                 185                 190
   Gly Asn Asp Lys Gly Glu Leu Val Val Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Thr Cys Asp Pro Arg Thr Val
   225                 230                 235                 240
   Pro Asp Ala Arg Leu Leu Lys Ser Met Ser Tyr Gln Glu Ala Met Glu
                   245                 250                 255
   Leu Ser Tyr Phe Gly Ala Lys Val Leu His Pro Arg Thr Ile Thr Pro
               260                 265                 270
   Ile Ala Gln Phe Gln Ile Pro Cys Leu Ile Lys Asn Thr Ser Asn Pro
           275                 280                 285
   Gln Ala Pro Gly Thr Leu Ile Gly Lys Asp Ser Thr Asp Ala Asp Met
       290                 295                 300
   Pro Val Lys Gly Ile Thr Asn Leu Asn Asn Met Ala Met Ile Asn Val
   305                 310                 315                 320
   Ser Gly Pro Gly Met Lys Gly Met Val Gly Met Ala Ala Arg Val Phe
                   325                 330                 335
   Ala Val Met Ser Arg Ala Gly Ile Ser Val Val Leu Ile Thr Gln Ser
               340                 345                 350
   Ser Ser Glu Tyr Ser Ile Ser Phe Cys Val Pro Gln Gly Glu Leu Gln
           355                 360                 365
   Arg Ala Arg Arg Ala Leu Glu Glu Glu Phe Tyr Leu Glu Leu Lys Asp
       370                 375                 380
   Gly Val Leu Asp Pro Leu Asp Val Met Glu Arg Leu Ala Ile Ile Ser
   385                 390                 395                 400
   Val Val Gly Asp Gly Met Arg Thr Leu Arg Gly Ile Ser Ala Arg Phe
                   405                 410                 415
   Phe Ser Ala Leu Ala Arg Ala Asn Ile Asn Ile Val Ala Ile Ala Gln
               420                 425                 430
   Gly Ser Ser Glu Arg Ser Ile Ser Val Val Val Ser Asn Asp Ser Ala
           435                 440                 445
   Thr Thr Gly Val Arg Val Ser His Gln Met Leu Phe Asn Thr Asp Gln
       450                 455                 460
   Val Ile Glu Val Phe Val Ile Gly Val Gly Gly Val Gly Gly Ala Leu
   465                 470                 475                 480
   Ile Glu Gln Ile Tyr Arg Gln Gln Pro Trp Leu Lys Gln Lys His Ile
                   485                 490                 495
   Asp Leu Arg Val Cys Gly Ile Ala Asn Ser Arg Val Met Leu Thr Asn
               500                 505                 510
   Val His Gly Ile Ala Leu Asp Ser Trp Arg Asp Ala Leu Ala Gly Ala
           515                 520                 525
   Gln Glu Pro Phe Asn Leu Gly Arg Leu Ile Arg Leu Val Lys Glu Tyr
       530                 535                 540
   His Leu Leu Asn Pro Val Ile Val Asp Cys Thr Ser Ser Gln Ala Val
   545                 550                 555                 560
   Ala Asp Gln Tyr Val Asp Phe Leu Ala Asp Gly Phe His Val Val Thr
                   565                 570                 575
   Pro Asn Lys Lys Ala Asn Thr Ser Ser Met Asn Tyr Tyr Gln Gln Leu
               580                 585                 590
   Arg Ala Ala Ala Ala Gly Ser His Arg Lys Phe Leu Tyr Asp Thr Asn
           595                 600                 605
   Val Gly Ala Gly Leu Pro Val Ile Glu Asn Leu Gln Asn Leu Leu Asn
       610                 615                 620
   Ala Gly Asp Glu Leu Val Arg Phe Ser Gly Ile Leu Ser Gly Ser Leu
   625                 630                 635                 640
   Ser Phe Ile Phe Gly Lys Leu Asp Glu Gly Leu Ser Leu Ser Ala Ala
                   645                 650                 655
   Thr Leu Gln Ala Arg Ala Asn Gly Tyr Thr Glu Pro Asp Pro Arg Asp
               660                 665                 670
   Asp Leu Ser Gly Met Asp Val Ala Arg Lys Leu Leu Ile Leu Ala Arg
           675                 680                 685
   Glu Ala Gly Tyr Lys Leu Glu Leu Ser Asp Ile Glu Val Glu Pro Val
       690                 695                 700
   Leu Pro Pro Ser Phe Asp Ala Ser Gly Asp Val Asp Thr Phe Leu Ala
   705                 710                 715                 720
   Arg Leu Pro Glu Leu Asp Lys Glu Phe Ala Arg Asn Val Ala Asn Ala
                   725                 730                 735
   Ala Glu Gln Gly Lys Val Leu Arg Tyr Val Gly Leu Ile Asp Glu Gly
               740                 745                 750
   Arg Cys Lys Val Arg Ile Glu Ala Val Asp Gly Asn Asp Pro Leu Tyr
           755                 760                 765
   Lys Val Lys Asn Gly Glu Asn Ala Leu Ala Phe Tyr Ser Arg Tyr Tyr
       770                 775                 780
   Gln Pro Leu Pro Leu Val Leu Arg Gly Tyr Gly Ala Gly Asn Asp Val
   785                 790                 795                 800
   Thr Ala Ala Gly Val Phe Ala Asp Leu Leu Arg Thr Leu Ser Trp Lys
                   805                 810                 815
   Leu Gly Val
<210> 11
   <211> 28
   <212> DNA
   <213> Artificial
<220>
   <223> Primer tig-1
<220>
   <221> misc_feature
   <222> (1)..(28)
   <223> Primer mit der Erkennungssequenz für das Restriktionsenzym XbaI
<400> 11
   gctctagaaa gagttgaccg agcactgt 28
<210> 12
   <211> 28
   <212> DNA
   <213> Artificial
<220>
   <223> Primer tig-2
<220>
   <221> misc_feature
   <222> (1)..(28)
   <223> Primer mit der Erkennungssequenz für das Restriktionsenzym HindIII
<400> 12
   gcaagcttat tacgcctgct ggttcatc 28
<210> 13
   <211> 1355
   <212> DNA
   <213> Escherichia coli
<220>
   <221> primer_bind
   <222> (1)..(28)
   <223> Primer-Bindungsstelle für tig-1
<220>
   <221> CDS
   <222> (48)..(1346)
   <223> Kodierregion tig
<220>
   <221> primer_bind
   <222> (1328)..(1355)
   <223> Primer-Bindungsstelle für tig-2
<400> 13
   gctctagaaa gagttgaccg agcactgtga ttttttgagg taacaag atg caa gtt        56
                                                       Met Gln Val
                                                       1
   tca gtt gaa acc act caa ggc ctt ggc cgc cgt gta acg att act atc       104
   Ser Val Glu Thr Thr Gln Gly Leu Gly Arg Arg Val Thr Ile Thr Ile
       5                   10                  15
   gct gct gac agc atc gag acc gct gtt aaa agc gag ctg gtc aac gtt       152
   Ala Ala Asp Ser Ile Glu Thr Ala Val Lys Ser Glu Leu Val Asn Val
   20                  25                  30                  35
   gcg aaa aaa gta cgt att gac ggc ttc cgc aaa ggc aaa gtg cca atg       200
   Ala Lys Lys Val Arg Ile Asp Gly Phe Arg Lys Gly Lys Val Pro Met
                   40                  45                  50
   aat atc gtt gct cag cgt tat ggc gcg tct gta cgc cag gac gtt ctg       248
   Asn Ile Val Ala Gln Arg Tyr Gly Ala Ser Val Arg Gln Asp Val Leu
               55                  60                  65
   ggt gac ctg atg agc cgt aac ttc att gac gcc atc att aaa gaa aaa       296
   Gly Asp Leu Met Ser Arg Asn Phe Ile Asp Ala Ile Ile Lys Glu Lys
           70                  75                  80
   atc aat ccg gct ggc gca ccg act tat gtt ccg ggc gaa tac aag ctg       344
   Ile Asn Pro Ala Gly Ala Pro Thr Tyr Val Pro Gly Glu Tyr Lys Leu
       85                  90                  95
   ggt gaa gac ttc act tac tct gta gag ttt gaa gtt tat ccg gaa gtt       392
   Gly Glu Asp Phe Thr Tyr Ser Val Glu Phe Glu Val Tyr Pro Glu Val
   100                 105                 110                 115
   gaa ctg cag ggt ctg gaa gcg atc gaa gtt gaa aaa ccg atc gtt gaa       440
   Glu Leu Gln Gly Leu Glu Ala Ile Glu Val Glu Lys Pro Ile Val Glu
                   120                 125                 130
   gtg acc gac gct gac gtt gac ggc atg ctg gat act ctg cgt aaa cag       488
   Val Thr Asp Ala Asp Val Asp Gly Met Leu Asp Thr Leu Arg Lys Gln
               135                 140                 145
   cag gcg acc tgg aaa gaa aaa gac ggc gct gtt gaa gca gaa gac cgc       536
   Gln Ala Thr Trp Lys Glu Lys Asp Gly Ala Val Glu Ala Glu Asp Arg
           150                 155                 160
   gta acc atc gac ttc acc ggt tct gta gac ggc gaa gag ttc gaa ggc       584
   Val Thr Ile Asp Phe Thr Gly Ser Val Asp Gly Glu Glu Phe Glu Gly
       165                 170                 175
   ggt aaa gcg tct gat ttc gta ctg gcg atg ggc cag ggt cgt atg atc       632
   Gly Lys Ala Ser Asp Phe Val Leu Ala Met Gly Gln Gly Arg Met Ile
   180                 185                 190                 195
   ccg ggc ttt gaa gac ggt atc aaa ggc cac aaa gct ggc gaa gag ttc       680
   Pro Gly Phe Glu Asp Gly Ile Lys Gly His Lys Ala Gly Glu Glu Phe
                   200                 205                 210
   acc atc gac gtg acc ttc ccg gaa gaa tac cac gca gaa aac ctg aaa       728
   Thr Ile Asp Val Thr Phe Pro Glu Glu Tyr His Ala Glu Asn Leu Lys
               215                 220                 225
   ggt aaa gca gcg aaa ttc gct atc aac ctg aag aaa gtt gaa gag cgt       776
   Gly Lys Ala Ala Lys Phe Ala Ile Asn Leu Lys Lys Val Glu Glu Arg
           230                 235                 240
   gaa ctg ccg gaa ctg act gca gaa ttc atc aaa cgt ttc ggc gtt gaa      824
   Glu Leu Pro Glu Leu Thr Ala Glu Phe Ile Lys Arg Phe Gly Val Glu
       245                 250                 255
   gat ggt tcc gta gaa ggt ctg cgc gct gaa gtg cgt aaa aac atg gag      872
   Asp Gly Ser Val Glu Gly Leu Arg Ala Glu Val Arg Lys Asn Met Glu
   260                 265                 270                 275
   cgc gag ctg aag agc gcc atc cgt aac cgc gtt aag tct cag gcg atc      920
   Arg Glu Leu Lys Ser Ala Ile Arg Asn Arg Val Lys Ser Gln Ala Ile
                   280                 285                 290
   gaa ggt ctg gta aaa gct aac gac atc gac gta ccg gct gcg ctg atc      968
   Glu Gly Leu Val Lys Ala Asn Asp Ile Asp Val Pro Ala Ala Leu Ile
               295                 300                 305
   gac agc gaa atc gac gtt ctg cgt cgc cag gct gca cag cgt ttc ggt     1016
   Asp Ser Glu Ile Asp Val Leu Arg Arg Gln Ala Ala Gln Arg Phe Gly
           310                 315                 320
   ggc aac gaa aaa caa gct ctg gaa ctg ccg cgc gaa ctg ttc gaa gaa     1064
   Gly Asn Glu Lys Gln Ala Leu Glu Leu Pro Arg Glu Leu Phe Glu Glu
       325                 330                 335
   cag gct aaa cgc cgc gta gtt gtt ggc ctg ctg ctg ggc gaa gtt atc     1112
   Gln Ala Lys Arg Arg Val Val Val Gly Leu Leu Leu Gly Glu Val Ile
   340                 345                 350                 355
   cgc acc aac gag ctg aaa gct gac gaa gag cgc gtg aaa ggc ctg atc     1160
   Arg Thr Asn Glu Leu Lys Ala Asp Glu Glu Arg Val Lys Gly Leu Ile
                   360                 365                 370
   gaa gag atg gct tct gcg tac gaa gat ccg aaa gaa gtt atc gag ttc     1208
   Glu Glu Met Ala Ser Ala Tyr Glu Asp Pro Lys Glu Val Ile Glu Phe
               375                 380                 385
   tac agc aaa aac aaa gaa ctg atg gac aac atg cgc aat gtt gct ctg     1256
   Tyr Ser Lys Asn Lys Glu Leu Met Asp Asn Met Arg Asn Val Ala Leu
           390                 395                 400
   gaa gaa cag gct gtt gaa gct gta ctg gcg aaa gcg aaa gtg act gaa     1304
   Glu Glu Gln Ala Val Glu Ala Val Leu Ala Lys Ala Lys Val Thr Glu
       405                 410                 415
   aaa gaa acc act ttc aac gag ctg atg aac cag cag gcg taa taagcttgc   1355
   Lys Glu Thr Thr Phe Asn Glu Leu Met Asn Gln Gln Ala
   420                 425                 430
<210> 14
   <211> 432
   <212> PRT
   <213> Escherichia coli
<400> 14
   Met Gln Val Ser Val Glu Thr Thr Gln Gly Leu Gly Arg Arg Val Thr
   1               5                   10                  15
   Ile Thr Ile Ala Ala Asp Ser Ile Glu Thr Ala Val Lys Ser Glu Leu
               20                  25                  30
   Val Asn Val Ala Lys Lys Val Arg Ile Asp Gly Phe Arg Lys Gly Lys
           35                  40                  45
   Val Pro Met Asn Ile Val Ala Gln Arg Tyr Gly Ala Ser Val Arg Gln
       50                  55                  60
   Asp Val Leu Gly Asp Leu Met Ser Arg Asn Phe Ile Asp Ala Ile Ile
   65                  70                  75                  80
   Lys Glu Lys Ile Asn Pro Ala Gly Ala Pro Thr Tyr Val Pro Gly Glu
                   85                  90                  95
   Tyr Lys Leu Gly Glu Asp Phe Thr Tyr Ser Val Glu Phe Glu Val Tyr
               100                 105                 110
   Pro Glu Val Glu Leu Gln Gly Leu Glu Ala Ile Glu Val Glu Lys Pro
           115                 120                 125
   Ile Val Glu Val Thr Asp Ala Asp Val Asp Gly Met Leu Asp Thr Leu
       130                 135                 140
   Arg Lys Gln Gln Ala Thr Trp Lys Glu Lys Asp Gly Ala Val Glu Ala
   145                 150                 155                 160
   Glu Asp Arg Val Thr Ile Asp Phe Thr Gly Ser Val Asp Gly Glu Glu
                   165                 170                 175
   Phe Glu Gly Gly Lys Ala Ser Asp Phe Val Leu Ala Met Gly Gln Gly
               180                 185                 190
   Arg Met Ile Pro Gly Phe Glu Asp Gly Ile Lys Gly His Lys Ala Gly
           195                 200                 205
   Glu Glu Phe Thr Ile Asp Val Thr Phe Pro Glu Glu Tyr His Ala Glu
       210                 215                 220
   Asn Leu Lys Gly Lys Ala Ala Lys Phe Ala Ile Asn Leu Lys Lys Val
   225                 230                 235                 240
   Glu Glu Arg Glu Leu Pro Glu Leu Thr Ala Glu Phe Ile Lys Arg Phe
                   245                 250                 255
   Gly Val Glu Asp Gly Ser Val Glu Gly Leu Arg Ala Glu Val Arg Lys
               260                 265                 270
   Asn Met Glu Arg Glu Leu Lys Ser Ala Ile Arg Asn Arg Val Lys Ser
           275                 280                 285
   Gln Ala Ile Glu Gly Leu Val Lys Ala Asn Asp Ile Asp Val Pro Ala
       290                 295                 300
   Ala Leu Ile Asp Ser Glu Ile Asp Val Leu Arg Arg Gln Ala Ala Gln
   305                 310                 315                 320
   Arg Phe Gly Gly Asn Glu Lys Gln Ala Leu Glu Leu Pro Arg Glu Leu
                   325                 330                 335
   Phe Glu Glu Gln Ala Lys Arg Arg Val Val Val Gly Leu Leu Leu Gly
               340                 345                 350
   Glu Val Ile Arg Thr Asn Glu Leu Lys Ala Asp Glu Glu Arg Val Lys
           355                 360                 365
   Gly Leu Ile Glu Glu Met Ala Ser Ala Tyr Glu Asp Pro Lys Glu Val
       370                 375                 380
   Ile Glu Phe Tyr Ser Lys Asn Lys Glu Leu Met Asp Asn Met Arg Asn
   385                 390                 395                 400
   Val Ala Leu Glu Glu Gln Ala Val Glu Ala Val Leu Ala Lys Ala Lys
                   405                 410                 415
   Val Thr Glu Lys Glu Thr Thr Phe Asn Glu Leu Met Asn Gln Gln Ala
               420                 425                 430

## Beschreibung

Diese Erfindung betrifft ein Verfahren zur fermentativen Herstellung der L-Aminosäuren L-Threonin und L-Tryptophan, unter Verwendung von rekombinanten Mikroorganismen der Familie Enterobacteriaceae, in denen das tig-Gen, welches für den Triggerfaktor TF kodiert, überexprimiert wird, und diese Mikroorganismen.

### Stand der Technik

L-Aminosäuren, insbesondere L-Threonin, L-Tryptophan, L-Lysin, L-Valin, L-Isoleucin und L-Homoserin, finden in der Humanmedizin und in der pharmazeutischen Industrie, in der Lebensmittelindustrie und in der Tierernährung Anwendung.

Es ist bekannt, dass L-Aminosäuren durch Fermentation von Stämmen der Enterobacteriaceae, insbesondere Escherichia coli (E. coli) und Serratia marcescens, hergestellt werden.

In EP1013765A wird ein Verfahren zur Herstellung der Aminosäuren L-Threonin und L-Homoserin durch Fermentation von Mikroorganismen der Familie Enterobacteriaceae offenbart, in welchem die Gene rhtB und rhtC überexprimiert werden.

In EP1715055A wird ein Verfahren zur Herstellung der Aminosäuren L-Threonin und L-Tryptophan durch Fermentation von Mikroorganismen der Familie Enterobacteriaceae offenbart, in welchem mindestens eines der Gene des Glycerin-Stoffwechsels überexprimiert wird.

Wegen der großen Bedeutung wird ständig an der Verbesserung der Herstellverfahren gearbeitet. Verfahrensverbesserungen können fermentationstechnische Maßnahmen, wie z.B. Rührung und Versorgung mit Sauerstoff, oder die Zusammensetzung der Nährmedien wie zum Beispiel die Auswahl des verwendeten Zuckers oder die Zuckerkonzentration während der Fermentation, oder die Aufarbeitung zur Produktform, durch z.B. Ionenaustauschchromatographie, oder die intrinsischen Leistungseigenschaften des Mikroorganismus selbst betreffen.

In Wildtypstämmen verhindern strikte Regulationsmechanismen die über den Eigenbedarf hinausgehende Produktion von Stoffwechselprodukten wie Aminosäuren und deren Abgabe ins Medium. Die Konstruktion von aus Herstellersicht Aminosäure überproduzierenden Stämmen erfordert deshalb, diese Stoffwechselregulationen zu überwinden.

Zur Beseitigung der Kontrollmechanismen und Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. das Threonin-Analogon α-Amino-β-Hydroxyvaleriansäure (AHV) oder auxotroph für regulatorisch bedeutsame Metabolite sind und L-Aminosäuren wie z.B. L-Threonin produzieren. Resistenz gegen das Tryptophan-Analogon 5-Methyl-DL-Tryptophan (5-MT) zeichnet zum Beispiel einen Stamm aus, der L-Tryptophan produziert.

Seit einigen Jahren werden ebenfalls Methoden der rekombinanten DNA-Technik zur gezielten Stammverbesserung L-Aminosäuren produzierender Stämme der Familie Enterobacteriaceae eingesetzt, indem man zum Beispiel einzelne Aminosäure-Biosynthesegene amplifiziert oder die Eigenschaften spezieller Gene verändert und die Auswirkung auf die Produktion untersucht. Zusammenfassende Informationen zur Zell- und Molekularbiologie von Escherichia coli und Salmonella sind in Neidhardt (ed): Escherichia coli and Salmonella, Cellular and Molecular Biology, 2nd edition, ASM Press, Washington, D.C., USA, (1996) zu finden. Eine Zusammenfassung zum Stoffwechsel und zur Produktion von L-Threonin ist veröffentlicht durch Debabov (Advances in Biochemical Engineering Vol.79, 113-136 (2003)).

Das Genprodukt des tig-Gens, der Triggerfaktor (TF; FK506-binding protein(FKBP)-type peptidyl-prolyl cis-trans isomerase), ist eines der drei wichtigsten Chaperone (zusammen mit DnaK und GroEL), die im Rahmen der Zellteilung bei der Faltung und Oligomerisierung von neusynthetisierten cytosolischen Proteinen zusammenarbeiten (Gragerov et al., Proceedings of the National Academy of Sciences of the United States of America 89(21):10341-4 (1992); Hartl und Hayer-Hartl, Science 295:1852-8 (2002)). TF wirkt auf einen Großteil der entstehenden, hauptsächlich kurzkettigen Polypeptide am Ribosom ein (Teter et al., Cell 97(6):755-65 (1999); Deuerling et al., Nature 400(6745):693-699 (1999); Tomic et al., FEBS Letters 580(1):72-76 (2006)), in dem es an den Proteinen L23/L29 am Polypeptidausgang bindet (Kramer et al., Nature 419(6903):171-4 (2002); Maier et al., Current Opinion in Structural Biology 15(2):204-12 (2005)) und dort, vermittelt durch die C-terminale Domäne, ATP-unabhängige Chaperone-Funktionen ausführt (Merz et al., Journal of Biological Chemistry 281(42):31963-71 (2006); Scholz et al., EMBO Journal 16(1):54-8 (1997)), wohingegen die durch die zentrale Domäne vermittelte cis/trans Isomerase Aktivität nicht essentiell für die korrekte Faltung der Polypeptide ist (Kramer et al., Journal of Biological Chemistry 279 (14):14165-70 (2004)).

Die Nukleotidsequenz der Wildform des für den Triggerfaktor TF von Escherichia coli kodierenden Gens wurde von Guthrie und Wickner (Journal of Bacteriology, 172(10), 5555-5562 (1990)) bestimmt und ist in der Datenbank des National Center for Biotechnology Information (NCBI) der National Library of Medicin (Bethesda, MD, USA) unter den Zugangsnummern (accession number) M34066 und U00096 (Region: 454357-455655) allgemein verfügbar.

### Aufgabe der Erfindung

Die Erfinder haben sich die Aufgabe gestellt, neue Maßnahmen zur Herstellung erhöhter Mengen der L-Aminosäuren L-Threonin und L-Tryptophan bereitzustellen.

### Beschreibung der Erfindung

Gegenstand der Erfindung sind rekombinante L-Threonin oder L-Tryptophan produzierende Mikroorganismen der Familie Enterobacteriaceae, die ein überexprimiertes tig-Gen enthalten, welches für den Triggerfaktor TF kodiert, wobei die für die Maßnahmen der Überexpression des tig-Gens eingesetzten Elternstämme bereits die Fähigkeit besitzen, mindestens 0,25 g/l L-Threonin oder L-Tryptophan in maximal 120 Stunden in der Zelle und/oder im Nährmedium beziehungsweise der Fermentationsbrühe anzureichern, wobei
a) die Mikroorganismen durch Transformation, Transduktion, Konjugation oder eine Kombination dieser Methoden hergestellt werden, mit einem Vektor, der das tig-Gen, ein Allel dieses Gens oder Teile davon, die die Aktivität des Triggerfaktors TF aufweisen, und gegebenenfalls einen Promotor enthält; und/oder
b) in den Mikroorganismen die Kopienzahl des tig-Gens oder der Allele im Vergleich zu den für das tig-Gen nicht rekombinanten Mikroorganismen um mindestens 1 erhöht vorliegt; und/oder
c) man zur Erzielung der Verstärkung die Promotor- und Regulationsregion oder die Ribosomenbindungsstelle stromaufwärts des tig-Gens mutiert oder Expressionskassetten oder Promotoren stromaufwärts des tig-Gens einbaut.

Zu diesen Mikroorganismen gehören insbesondere Mikroorganismen der Familie Enterobacteriaceae, in denen ein Polynukleotid verstärkt wird, das für ein Polypeptid kodiert, dessen Aminosäuresequenz zu mindestens 70% oder zu mindestens 80% oder mindestens 90%, insbesondere zu mindestens 95%, bevorzugt zu mindestens 98% oder mindestens 99%, besonders bevorzugt zu 99,6% und ganz besonders bevorzugt zu 100% identisch ist mit einer Aminosäuresequenz, ausgewählt aus der Gruppe SEQ ID No. 2, SEQ ID No. 4 und SEQ ID No. 6, bevorzugt SEQ ID No. 2, wobei das Polypeptid die Aktivität des Triggerfaktors TF besitzt.

Die genannten Mikroorganismen enthalten isolierte Polynukleotide, ausgewählt aus der Gruppe:
a) Polynukleotid mit einer Nukleotidsequenz, ausgewählt aus SEQ ID No. 1, SEQ ID No. 3 oder SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen bevorzugt durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für den Triggerfaktor TF kodieren.

Als vor der erfindungsgemäßen Verstärkung oder Überexpression L-Aminosäure produzierende Mikroorganismen zählen dabei nicht die Wildtypstämme und häufig benutzte Laborstämme wie unter anderen DH5α, DH5αmcr, W3110, MG1655, MC4100, Y1089, H560 und MM152.

Bevorzugt werden die beschriebenen Mikroorganismen eingesetzt.

Werden im folgenden L-Aminosäuren oder Aminosäuren erwähnt, sind damit eine oder mehrere Aminosäuren einschließlich ihrer Salze, ausgewählt aus L-Threonin und L-Tryptophan gemeint.

Gegebenenfalls kann es von Vorteil sein, nur eine gemäßigte Erhöhung der intrazellulären Aktivität oder Konzentration eines oder mehrerer Enzyme oder Proteine in einem Mikroorganismus durchzuführen, da eine zu starke Erhöhung zum Beispiel zu einer fehlerhaften Zellteilung oder veränderter Zellmorphologie bis hin zur Toxizität führen kann (Guthrie und Wickner, Journal of Bacteriology 172(10):5555-5562 (1990); Genevaux P. et al.; EMBO Reports 5(2): 195-200 (2004)).

Der Begriff "gemäßigte Erhöhung" beschreibt die Erhöhung der intrazellulären Aktivität oder Konzentration des entsprechenden Proteins um höchstens das 10-fache, das 8-fache, das 6-fache, das 4-fache, das 3-fache, das 2-fache oder das 1,5-fache bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm. Als nicht rekombinanter Mikroorganismus oder Elternstamm (parent strain) wird der Mikroorganismus verstanden, an dem die erfindungsgemäße Verstärkung oder Überexpression durchgeführt wird.

Die Kopienzahl des erfindungsgemäßen tig-Gens wird bevorzugt um höchstens acht (8) Kopien erhöht, besonders bevorzugt um höchstens vier (4) Kopien. Eine solche gemäßigte Expression ist zum Beispiel erreichbar durch die Verwendung von Plasmiden mit lacI^{Q}-Allel-kontrollierten Genen (Glascock und Weickert, Gene 223, 221-231 (1998)) ohne Induktion durch IPTG-Zugabe, welches zu einer sehr schwachen Expression des ohne eigenen Promotor klonierten tig-Gens führt. Der Transkriptionsstart des tig-Gens wurde 139 Basenpaare vor dem ATG-Startkodon lokalisiert (Aldea at al., EMBO Journal 8(12): 3923-3931 (1989)) und nicht zusammen mit der für den Trigger Faktor kodierenden Nukleotidsequenz auf das Plasmid übertragen.

Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich, Angewandte Chemie 38: 2630-2647 (1999)) bestimmt werden. Ein Gen oder Allel ist chemisch ein Polynukleotid. Ein anderer Begriff hierfür ist Nukleinsäure, insbesondere Desoxyribonukleinsäure.

Die Begriffe Polypeptid und Protein sind gegenseitig austauschbar.

Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure. Gegenstand der Erfindung ist ebenfalls ein Verfahren zur Herstellung der L-Aminosäuren L-Threonin oder L-Tryptophan durch Fermentation der erfindungsgemäßen rekombinanten Mikroorganismen der Familie Enterobacteriaceae, dadurch gekennzeichnet, dass man
a) die L-Threonin oder L-Tryptophan produzierenden Mikroorganismen, die ein überexprimiertes tig-Gen enthalten, welches für den Triggerfaktor TF kodiert,in einem Medium kultiviert unter Bedingungen, bei denen die gewünschte L-Aminosäure L-Threonin oder L-Tryptophan im Medium oder in den Zellen angereichert wird, und
b) die gewünschte L-Aminosäure L-Threonin oder L-Tryptophan isoliert, wobei gegebenenfalls weitere Bestandteile der Fermentationsbrühe und/oder die Biomasse in ihrer Gesamtheit oder Anteilen (≥ 0 bis 100 %) im isolierten Produkt verbleiben oder vollständig entfernt werden.

Die rekombinanten Mikroorganismen mit einem überexprimierten tig-Gen, die Gegenstand der vorliegenden Erfindung sind, können L-Aminosäuren aus Glucose, Saccharose, Laktose, Fructose, Maltose, Melasse, gegebenenfalls Stärke, gegebenenfalls Cellulose oder aus Glycerin und Ethanol, gegebenenfalls auch aus Mischungen, herstellen. Es handelt sich um Vertreter der Familie Enterobacteriaceae, ausgewählt aus den Gattungen Escherichia, Erwinia, Providencia und Serratia. Die Gattungen Escherichia und Serratia werden bevorzugt. Bei der Gattung Escherichia ist insbesondere die Art Escherichia coli und bei der Gattung Serratia insbesondere die Art Serratia marcescens zu nennen.

In einer bevorzugten Ausführungsform werden die rekombinanten Mikroorganismen durch Transformation, Transduktion oder Konjugation, oder einer Kombination dieser Methoden, mit einem Vektor erzeugt, der das tig-Gen, ein Allel dieses Gens oder Teile davon, die die Aktivität des Triggerfaktors TF aufweisen, und gegebenenfalls einen Promotor enthält. Bei diesem Promotor kann es sich um den durch verstärkende Mutation aus der eigenen, upstream des Gens oder ORFs befindlichen regulatorischen Sequenz hervorgegangenen Promotor handeln, oder es wurde ein effizienter Promotor mit dem Gen oder ORF fusioniert.

Zur Herstellung der L-Aminosäure-anreichernden Stämme der Familie Enterbacteriaceae, welche ein verstärktes tig-Gen enthalten, werden Stämme verwendet (Ausgangsstämme bzw. Elternstämme), die bereits die Fähigkeit besitzen, die gewünschte L-Aminosäure in der Zelle anzureichern und/oder in das sie umgebende Nährmedium auszuscheiden bzw. in der Fermentationsbrühe zu akkumulieren. Hierfür kann auch der Ausdruck "produzieren"
verwendet werden. Die für die Maßnahmen der Erfindung eingesetzten Stämme besitzen die Fähigkeit ≥ (mindestens) 0,25 g/l, insbesondere ≥ 0,5 g/l, ≥ 1,0 g/l, ≥ 1,5 g/l, ≥ 2,0 g/l, ≥ 4 g/l der 10 g/l an L-Aminosäure in ≤ (maximal) 120 Stunden, insbesondere ≤ 96 Stunden, ≤ 48 Stunden, ≤ 36 Stunden, ≤ 24 Stunden oder ≤ 12 Stunden in der Zelle und/oder im Nährmedium bzw. der Fermentationsbrühe anzureichern bzw. zu akkumulieren. Hierbei kann es sich um Stämme handeln, die durch Mutagenese und Selektion, durch rekombinante DNA-Techniken oder durch eine Kombination beider Methoden hergestellt wurden.

Die genannten L-Aminosäuren-ausscheidenden Stämme produzieren eine oder mehrere, bevorzugt eine oder im Wesentlichen eine Aminosäure ausgewählt aus der Gruppe L-Threonin und L-Tryptophan.Der Begriff L-Aminosäure oder Aminosäure umfasst auch deren Salze.

Der Begriff " eine oder im Wesentlichen eine Aminosäure" berücksichtigt, dass zusätzlich zu der gewünschten L-Aminosäure eine oder mehrere weitere L-Aminosäuren (Nebenaminosäuren) produziert werden können. Der Anteil dieser Nebenaminosäuren beträgt ≥ 0 bis maximal 40%, bevorzugt ≥ 0 bis maximal 20%, besonders bevorzugt ≥ 0 bis maximal 10% und ganz besonders bevorzugt ≥ 0 bis maximal 5% bezogen auf die Menge der gewünschten L-Aminosäure.

Als zum Elternstamm geeignete, insbesondere L-Threonin produzierende bzw. ausscheidende Stämme der Gattung Escherichia, insbesondere der Art Escherichia coli sind beispielsweise zu nennen:
- Escherichia coli H4581 (EP 0 301 572)
- Escherichia coli KY10935 (Bioscience Biotechnology and Biochemistry 61(11):1877-1882 (1997)
- Escherichia coli VNIIgenetika MG442 (US-A-4278,765)
- Escherichia coli VNIIgenetika M1 (US-A-4,321,325)
- Escherichia coli VNIIgenetika 472T23 (US-A-5,631,157)
- Escherichia coli TH 14.97 (WO 02/26993)
- Escherichia coli TH 21.97 (WO 02/26993)
- Escherichia coli BKIIM B-3996 (US-A-5,175,107)
- Escherichia coli BKIIM B-3996ΔtdhΔpckA/pVIC40 (WO 02/29080)
- Escherichia coli kat 13 (WO 98/04715)
- Escherichia coli Kat 69.9 (WO 02/26993)
- Escherichia coli KCCM-10132 (WO 00/09660)
- Escherichia coli KCCM-10168 (WO 01/14525)
- Escherichia coli KCCM-10133 (WO 00/09661)

Als zum Elternstamm geeignete L-Threonin produzierende bzw. ausscheidende Stämme der Gattung Serratia, insbesondere der Art Serratia marcescens sind beispielsweise zu nennen:
- Serratia marcescens HNr21 (Applied and Environmental Microbiology 38(6): 1045-1051 (1979))
- Serratia marcescens TLr156 (Gene 57(2-3): 151-158 (1987))
- Serratia marcescens T-2000 (AppliedBiochemistry and Biotechnology 37(3): 255-265 (1992))

L-Threonin produzierende oder ausscheidende Stämme aus der Familie der Enterobacteriaceae besitzen bevorzugt, unter anderem, ein oder mehrere der genetischen bzw. phänotypischen Merkmale ausgewählt aus der Gruppe: Resistenz gegen α-Amino-β-Hydroxyvaleriansäure, Resistenz gegen Thialysin, Resistenz gegen Ethionin, Resistenz gegen α-Methylserin, Resistenz gegen Diaminobernsteinsäure, Resistenz gegen α-Aminobuttersäure, Resistenz gegen Borrelidin, Resistenz gegen Cyclopentan-Carboxylsäure, Resistenz gegen Rifampicin, Resistenz gegen Valin-Analoga wie beispielsweise Valinhydroxamat, Resistenz gegen Purinanaloga wie beispielsweise 6-Dimethylaminopurin, Bedürftigkeit für L-Methionin, gegebenenfalls partielle und kompensierbare Bedürftigkeit für L-Isoleucin, Bedürftigkeit für meso-Diaminopimelinsäure, Auxotrophie bezüglich Threonin-haltiger Dipeptide, Resistenz gegen L-Threonin, Resistenz gegen Threonin-Raffinat, Resistenz gegen L-Homoserin, Resistenz gegen L-Lysin, Resistenz gegen L-Methionin, Resistenz gegen L-Glutaminsäure, Resistenz gegen L-Aspartat, Resistenz gegen L-Leucin, Resistenz gegen L-Phenylalanin, Resistenz gegen L-Serin, Resistenz gegen L-Cystein, Resistenz gegen L-Valin, Empfindlichkeit gegenüber Fluoropyruvat, defekte Threonin-Dehydrogenase, gegebenenfalls Fähigkeit zur Saccharose-Verwertung, Verstärkung des Threonin-Operons, Verstärkung der Homoserin-Dehydrogenase I-Aspartatkinase I bevorzugt der feed back resistenten Form, Verstärkung der Homoserinkinase, Verstärkung der Threoninsynthase, Verstärkung der Aspartatkinase, gegebenenfalls der feed back resistenten Form, Verstärkung der Aspartatsemialdehyd-Dehydrogenase, Verstärkung der Phosphoenolpyruvat-Carboxylase, gegebenenfalls der feed back resistenten Form, Verstärkung der Phosphoenolpyruvat-Synthase, Verstärkung der Transhydrogenase, Verstärkung des RhtB-Genproduktes, Verstärkung des RhtC-Genproduktes, Verstärkung des YfiK-Genproduktes, Verstärkung einer Pyruvat-Carboxylase, und Abschwächung der Essigsäurebildung.

Als Elternstamm geeignete, L-Tryptophan produzierende bzw. ausscheidende Stämme der Gattung Escherichia, insbesondere der Art Escherichia coli sind beispielsweise zu nennen:
- Escherichia coli JP4735/pMU3028 (US5,756,345)
- Escherichia coli JP6015/pMU91 (US5,756,345)
- Escherichia coli SV164(pGH5) (WO94/08031)
- E. coli AGX17(pGX44) (NRRL B-12263) (US4,371,614)
- E. coli AGX6(pGX50)aroP (NRRL B-12264) (US4,371,614)
- Escherichia coli AGX17/pGX50,pACKG4-pps (WO97/08333)
- Escherichia coli ATCC 31743 (CA1182409)
- E. coli C534/pD2310,pDM136 (ATCC 39795) (WO87/01130)
- Escherichia coli JB102/p5LRPS2 (US5,939,295).

L-Tryptophan produzierende oder ausscheidende Stämme aus der Familie der Enterobacteriaceae besitzen bevorzugt, unter anderen, ein oder mehrere der genetischen bzw. phänotypischen Merkmale ausgewählt aus der Gruppe: Resistenz gegen 5-Methyl-DL-Tryptophan, Resistenz gegen 5-Fluoro-Tryptophan, Resistenz gegen 4-Methyl-DL-Tryptophan, Resistenz gegen 6-Methyl-DL-Tryptophan, Resistenz gegen 4-Fluoro-Tryptophan, Resistenz gegen 6-Fluoro-Tryptophan, Resistenz gegen Anthranilat, Resistenz gegen Tryptazan, Resistenz gegen Indol, Resistenz gegen Indol-Acrylsäure, Bedürftigkeit für Phenylalanin, Bedürftigkeit für Tyrosin, gegebenenfalls Fähigkeit zur Saccharose-Verwertung, Verstärkung des Tryptophan-Operons, bevorzugt der Anthranilat-Synthase bevorzugt der feed back resistenten Form, eine teilweise defekte Tryptophanyl-tRNA-Synthase, eine abgeschwächte Tryptophan-Aufnahme, eine defekte Tryptophanase, inaktivierte Repressorproteine, Verstärkung der Serin-Biosynthese, Verstärkung der Phosphoenolpyruvat-Synthese, Verstärkung der D-Erythrose-4-Phosphat-Synthese, Verstärkung der 3-deoxy-D-arabino-heptulosonat-7-Phosphat(DHAP)-Synthese, Verstärkung der Chorismat-Biosynthese.

Bei den der Erfindung zugrunde liegenden Arbeiten wurde gefunden, dass Mikroorganismen der Familie Enterobacteriaceae nach Überexpression des Gens tig, oder dessen Allelen, vermehrt die L-Aminosäuren L-Threonin und L-Tryptophan produzieren und in der Zelle oder im Medium anreichern.

Die Nukleotidsequenzen der Gene oder offenen Leserahmen (ORF) von Escherichia coli gehören zum Stand der Technik und können der von Blattner et al. (Science 277: 1453-1462 (1997)) publizierten Genomsequenz von Escherichia coli entnommen werden. Es ist bekannt, dass durch wirtseigene Enzyme (Methionin-Aminopeptidase) die N-terminale Aminosäure Methionin abgespalten werden kann.

Aus den ebenfalls zur Familie Enterobacteriaceae gehörenden Salmonella enterica und Erwinia carotovora ist die Nukleotidsequenz für das tig-Gen ebenso bekannt (Accession No.: NC_003198 (REGION: (493813-495111) und Accession No.: NC_004547 (REGION: (1299536-1300840)). Weitere Nukleotidsequenzen für das tig-Gen findet man aus folgenden Enterobacteriaceae: Shigella flexneri (Accession No.: CP000266, AE005674, AE014073); Shigella boydii (Accession No.: CP000036); Shigella dysenteriae (Accession No.: CP000034); Shigella sonnei (Accession No.: CP000038); Salmonella typhimurium (Accession No.: AE008716); Sodalis glossinidius (Accession No.: AP008232); Photorhabdus luminescens (Accession No.: BX571872).

Das tig-Gen von Escherichia coli K12 wird unter anderem durch folgende Angaben beschrieben:
- Bezeichnung:: Trigger Faktor
- Funktion:: Chaperon, beteiligt am Protein-Export durch Stabilisierung von Proteinen in einer Translokations-Form, Peptidyl-prolyl cis/trans Isomerase
- Referenz:: Crooke E. und Wickner W.; Proceedings of the National Academy of Sciences of the United States of America 84(15): 5216-5220 (1987)
Lee HC. und Bernstein HD.; The Journal of biological chemistry 277(45): 43527-43535 (2002)
Genevaux P. et al.; EMBO reports 5(2): 195-200 (2004)
Kramer et al.; The Journal of biological chemistry 279(14): 14165-14170 (2004)
- Accession No.:: U00096 (Region: (454357-455655))

Das kodierte Polypeptid besitzt eine Länge von 432 Aminosäuren.
- Alternativer Genname:: b0436, EG11003

Die Nukleinsäuresequenzen können den Datenbanken des National Center for Biotechnology Information (NCBI) der National Library of Medicine (Bethesda, MD, USA), der Nukleotidsequenz-Datenbank der European Molecular Biologies Laboratories (EMBL, Heidelberg, Deutschland bzw. Cambridge, UK) oder der DNA Datenbank von Japan (DDBJ, Mishima, Japan) entnommen werden.

Der besseren Übersichtlichkeit halber sind die bekannte Sequenz zum tig-Gen von Escherichia coli unter der SEQ ID No. 1 und die bekannten Sequenzen zum tig-Gen von Salmonella enterica und Erwinia carotovora unter den SEQ ID No. 3 und SEQ ID No. 5 dargestellt. Die von diesen Leserahmen kodierten Proteine sind als SEQ ID No. 2, SEQ ID No. 4 und SEQ ID No. 6 dargestellt.

Die in den angegebenen Textstellen beschriebenen Gene oder offenen Leserahmen können erfindungsgemäß verwendet werden. Weiterhin können Allele der Gene oder offene Leserahmen verwendet werden, die sich aus der Degeneriertheit des genetischen Codes oder durch funktionsneutrale Sinnmutationen ("sense mutations") ergeben. Die Verwendung endogener Gene bzw. endogener offener Leserahmen wird bevorzugt.

Unter "endogenen Genen" oder "endogenen Nukleotidsequenzen" versteht man die in der Population einer Art vorhandenen Gene oder offene Leserahmen oder Allele beziehungsweise Nukleotidsequenzen.

Zu den Allelen des tig-Gens, welche funktionsneutrale Sinnmutationen enthalten, zählen unter anderem solche, die zu höchstens 30 oder zu höchstens 20, bevorzugt zu höchstens 10 oder zu höchstens 5, ganz besonders bevorzugt zu höchstens 3 oder zu höchstens 2 oder zu mindestens einem konservativen Aminosäureaustausch in dem von ihnen kodierten Protein führen.

Bei den aromatischen Aminosäuren spricht man von konservativen Austauschen wenn Phenylalanin, Tryptophan und Tyrosin gegeneinander ausgetauscht werden. Bei den hydrophoben Aminosäuren spricht man von konservativen Austauschen wenn Leucin, Isoleucin und Valin gegeneinander ausgetauscht werden. Bei den polaren Aminosäuren spricht man von konservativen Austauschen wenn Glutamin und Asparagin gegeneinander ausgetauscht werden. Bei den basischen Aminosäuren spricht man von konservativen Austauschen wenn Arginin, Lysin und Histidin gegeneinander ausgetauscht werden. Bei den sauren Aminosäuren spricht man von konservativen Austauschen wenn Asparaginsäure und Glutaminsäure gegeneinander ausgetauscht werden. Bei den Hydroxyl-Gruppen enthaltenden Aminosäuren spricht man von konservativen Austauschen wenn Serin und Threonin gegeneinander ausgetauscht werden.

In gleicher Weise können auch solche Nukleotidsequenzen verwendet werden, die für Varianten der genannten Proteine kodieren, die zusätzlich am N- oder C-Terminus eine Verlängerung oder Verkürzung um mindestens eine (1) Aminosäure enthalten. Diese Verlängerung oder Verkürzung beträgt nicht mehr als 40, 30, 20, 10, 5, 3 oder 2 Aminosäuren oder Aminosäurereste.

Zu den geeigneten Allelen zählen auch solche, die für Proteine kodieren, in denen mindestens eine (1) Aminosäure eingefügt (Insertion) oder entfernt (Deletion) ist. Die maximale Anzahl derartige als Indels bezeichneter Veränderungen kann 2, 3, 5, 10 in keinem Falle aber mehr als 20 Aminosäuren betreffen.

Zu den geeigneten Allelen gehören ferner solche die durch Hybridisierung, insbesondere unter stringenten Bedingungen unter Verwendung von SEQ ID No. 1, SEQ ID No. 3 oder SEQ ID No. 5 oder Teilen davon insbesondere der Kodierregionen bzw. der dazu komplementären Sequenzen, erhältlich sind.

Anleitungen zur Identifizierung von DNA-Sequenzen mittels Hybridisierung findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen Sonde und Zielsequenz, d. h. die mit der Sonde behandelten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Sequenz der Sonde aufweisen. Solche Hybride sind weniger stabil und werden durch Waschen unter stringenten Bedingungen entfernt. Dies kann beispielsweise durch Senken der Salzkonzentration auf 2x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich die Salzkonzentration bis auf eine Konzentration entsprechend 0,2x SSC oder 0,1x SSC zu senken. Durch schrittweise Erhöhung der Hybridisierungstemperatur in Schritten von ca. 1 - 2°C von 50°C auf 68°C können Polynukleotidfragmente isoliert werden, die beispielsweise mindestens 70% oder mindestens 80% oder mindestens 90%, mindestens 91%, mindestens 92%, mindestens 93%, mindestens 94%, mindestens 95%, mindestens 96%, mindestens 97%, mindestens 98% oder mindestens 99% Identität zur Sequenz der eingesetzten Sonde bzw. zu den in SEQ ID No. 1, SEQ ID No. 3 oder SEQ ID No. 5 dargestellten Nukleotidsequenzen besitzen. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Zur Erzielung einer Überexpression kann beispielsweise die Kopienzahl der entsprechenden Gene oder offenen Leserahmen erhöht werden, oder es kann die Promotor- und Regulationsregion oder die Ribosomenbindungsstelle, die sich stromaufwärts des Strukturgens befindet, mutiert werden. In gleicher Weise wirken Expressionskassetten, die stromaufwärts des Strukturgens eingebaut werden. Durch induzierbare Promotoren ist es zusätzlich möglich, die Expression im Verlaufe der fermentativen L-Threonin-Produktion zu steigern, des weiteren vorteilhaft sein kann auch die Verwendung von Promotoren zur Genexpression, die eine andere zeitliche Genexpression ermöglicht. Auf der Ebene der translationalen Regulation der Genexpression ist es möglich, die Häufigkeit der Initiation (Anlagerung des Ribosoms an die m-RNA) oder die Geschwindigkeit der Elongation (Verlängerungsphase) zu erhöhen. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene, ORFs oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Methoden der Überexpression sind im Stand der Technik hinlänglich - beispielsweise bei Makrides et al. (Microbiological Reviews 60 (3), 512-538 (1996)) - beschrieben. Durch Verwendung von Vektoren wird die Kopienzahl um mindestens eine (1) Kopie erhöht. Als Vektoren können Plasmide wie beispielsweise in der US 5,538,873 beschrieben verwendet werden. Als Vektoren können ebenfalls Phagen, beispielsweise der Phage Mu, wie in der EP 0332448 beschrieben, oder der Phage lambda (A) verwendet werden. Eine Erhöhung der Kopienzahl kann auch dadurch erzielt werden, dass eine weitere Kopie in eine weitere Stelle des Chromosoms - beispielsweise in die att-site des Phagen A (Yu und Court, Gene 223, 77-81 (1998)) - eingebaut wird. In der US 5,939,307 wird beschrieben, dass durch Einbau von Expressionskassetten oder Promotoren wie beispielsweise tac-Promotor, trp-Promotor, lpp-Promotor oder P_{L}-Promotor und P_{R}-Promotor des Phagen A beispielsweise stromaufwärts des chromosomalen Threoninoperons eine Erhöhung der Expression erzielt werden konnte. In gleicher Weise können die Promotoren des Phagen T7, die gear-box-Promotoren, der nar-Promotor oder die Promotoren der Gene rrsG, rnpB, csrA, csrB, ompA, fusA, pepQ, rplX oder rpsG verwendet werden. Derartige Expressionskassetten oder Promotoren können auch verwendet werden um, wie in der EP 0 593 792 beschrieben, plasmidgebundene Gene zu überexprimieren. Durch Verwendung des lacI^{Q}-Allels lässt sich wiederum die Expression von lac-Promotor-Genfusionen kontrollieren (Glascock und Weickert, Gene 223, 221-231 (1998)), die Verwendung dieser Plasmide ohne Induktion durch IPTG-Zugabe führt zu einer sehr schwachen Expression von ohne eigenem Promotor klonierten Genen. Es ist weiterhin möglich, dass die Aktivität der Promotoren durch Modifikation ihrer Sequenz mittels einem oder mehreren Nukleotidaustauschen, durch Insertion (en)und/oder Deletion(en) erhöht ist. Der Austausch des Promotors gegen einen zum Beispiel in genomweit-vergleichenden Expressionsanalysen ermittelten Promotor mit gleichmäßig hoher Expression im Verlauf eines Fermentationsprozesses bewirkt eine gleichmäßige Verstärkung. Eine andere zeitliche Genexpression kann beispielsweise wie bei Walker et al. (Journal of Bacteriology 181: 1269-80 (1999)) beschrieben durch die Verwendung des Wachstumsphasen-abhängigen fis Promotors erreicht werden. Die Geschwindigkeit der Elongation wird durch die Codon-Verwendung beeinflusst, durch den Gebrauch von Codons für im Elternstamm (parent strain) häufig vorkommenden t-RNAs kann die Genexpression verstärkt werden. Desweiteren kann der Austausch eines Start-Codons zu dem in Escherichia coli mit 77% am häufigsten vorkommenden Codon ATG die Translation erheblich verbessern, da das Codon AUG zwei- bis dreimal effektiver ist als zum Beispiel die Codons GUG und UUG (Khudyakov et al., FEBS Letters 232(2):369-71 (1988); Reddy et al., Proceedings of the National Academy of Sciences of the USA 82(17):5656-60 (1985)). Auch die Sequenzumgebung des Start-Codons kann optimiert werden, da zusammenwirkende Effekte zwischen dem Start-Codon und den flankierenden Bereichen beschrieben sind (Stenstrom et al., Gene 273(2):259-65 (2001); Hui et al., EMBO Journal 3(3):623-9 (1984)).

Allgemeine Anleitungen hierzu findet der Fachmann unter anderem bei Chang und Cohen (Journal of Bacteriology 134: 1141-1156 (1978)), bei Hartley und Gregori (Gene 13: 347-353 (1981)), bei Amann und Brosius (Gene 40: 183-190 (1985)), bei de Broer et al. (Proceedings of the National Academy of Sciences of the United States of America 80: 21-25 (1983)), bei LaVallie et al. (BIO/TECHNOLOGY 11: 187-193 (1993)), in PCT/US97/13359, bei Llosa et al. (Plasmid 26: 222-224 (1991)), bei Quandt und Klipp (Gene 80: 161-169 (1989)), bei Hamilton et al. (Journal of Bacteriology 171: 4617-4622 (1989)), bei Jensen und Hammer (Biotechnology and Bioengineering 58: 191-195 (1998)) und in bekannten Lehrbüchern der Genetik und Molekularbiologie.

In Enterobacteriaceae replizierbare Plasmidvektoren wie z.B. von pACYC184 abgeleitete Kloniervektoren (Bartolome et al.; Gene 102: 75-78 (1991)), pTrc99A (Amann et al.; Gene 69: 301-315 (1988)) oder pSC101-Derivate (Vocke und Bastia; Proceedings of the National Academy of Sciences USA 80(21): 6557-6561 (1983)) können verwendet werden. In einem erfindungsgemäßen Verfahren kann man einen mit einem Plasmidvektor transformierten Stamm einsetzen, wobei der Plasmidvektor mindestens das tig-Gen, oder für dessen Genprodukt kodierende Nukleotidsequenzen oder Allele trägt.

Unter dem Begriff Transformation versteht man die Aufnahme einer isolierten Nukleinsäure durch einen Wirt (Mikroorganismus).

Zur Erzeugung von tig-Allelen, die in den für das erfindungsgemäße Verfahren eingesetzten Mikroorganismen zum Einsatz kommen, können unter anderem im Stand der Technik beschriebene Methoden zur gerichteten Mutagenese verwendet werden.

Es können Verfahren der ortsgerichteten Mutagenese unter Verwendung mutagener Oligonukleotide (T. A. Brown: Gentechnologie für Einsteiger, Spektrum Akademischer Verlag, Heidelberg, 1993) oder der Polymerase-Kettenreaktion (PCR), wie sie im Handbuch von Gait: Oligonukleotide synthesis: A Practical Approach (IRL Press, Oxford, UK, 1984) oder von Newton und Graham (PCR, Spektrum Akademischer Verlag, Heidelberg, 1994) beschrieben sind, verwendet werden. Die erzeugten Mutationen können durch DNA-Sequenzierung beispielsweise nach der Methode von Sanger et al. (Proceedings of the National Academy of Science USA 74 (12): 5463-5467 (1977)) bestimmt und überprüft werden.

Zur Konstruktion von Punktmutationen im tig-Gen kann zum Beispiel das Quick Change Site-Directed Mutagenesis Kit der Firma Stratagene (Amsterdam, Niederlande) verwendet werden. Bei Verwendung dieser Methoden wird das im Stand der Technik beschriebene tig-Gen ausgehend von isolierter Gesamt-DNA eines Wildtypstammes mit Hilfe der Polymerase-Kettenreaktion (PCR) amplifiziert, in geeignete Plasmidvektoren kloniert, und die DNA anschließend dem Mutageneseverfahren unterworfen. Mittels "GeneSOEing" (Gene Splicing by Overlap Extension, Horton, Molecular Biotechnology 3: 93-98 (1995)) können die Punktmutationen schon per PCR erhalten werden.

Die erzeugten tig-Allele können beispielsweise durch Transformation und das Verfahren des Gen- bzw. Allelaustausches in geeignete Stämme eingebaut werden.

Eine gebräuchliche Methode ist die von Hamilton et al. (Journal of Bacteriology 174, 4617 - 4622 (1989)) beschriebene Methode des Genaustauschs mit Hilfe eines konditional replizierenden pSC101-Derivates pMAK705 oder mit pKO3 (Link et al., Journal of Bacteriology 179 : 6228-6237). Andere im Stand der Technik beschriebene Methoden wie beispielsweise die von Martinez-Morales et al. (Journal of Bacteriology 1999, 7143-7148 (1999)) oder die von Boyd et al. (Journal of Bacteriology 182, 842-847 (2000)) können gleichfalls benutzt werden.

Es ist ebenfalls möglich die erzeugten tig-Allele durch Konjugation oder Transduktion in verschiedene Stämme zu überführen.

Die Mutationen von tig können ebenfalls in Expressionsplasmiden durchgeführt werden, so dass die resultierenden Proteine überproduziert werden können. Nähere Erläuterungen zu den Begriffen der Genetik und Molekularbiologie findet man in bekannten Lehrbüchern der Genetik und Molekularbiologie wie beispielsweise dem Lehrbuch von Birge (Bacterial and Bacteriophage Genetics, 4th ed., Springer Verlag, New York (USA), 2000) oder dem Lehrbuch von Berg, Tymoczko and Stryer (Biochemistry, 5±h ed., Freeman and Company, New York (USA), 2002) oder dem Handbuch von Sambrook et al. (Molekular Cloning, A Laboratory Manual, (3-Volume Set), Cold Spring Harbor Laboratory Press, Cold Spring Harbor (USA), 2001).

Weiterhin kann es für die Produktion von L-Aminosäuren, insbesondere L-Threonin, mit Stämmen der Familie Enterobacteriaceae vorteilhaft sein, zusätzlich zur Verstärkung des tig-Gens, ein oder mehrere Enzym(e) des bekannten Threonin-Biosyntheseweges oder Enzyme des anaplerotischen Stoffwechsels oder Enzyme für die Produktion von reduziertem Nicotinamid-Adenin-Dinukleotid-Phosphat oder Enzyme der Glykolyse oder PTS-Enzyme oder Enzyme des Schwefelstoffwechsels zu verstärken. Die Verwendung endogener Gene wird im Allgemeinen bevorzugt.

Ebenso kann es für die Produktion von L-Trytophan mit Stämmen der Familie Enterobacteriaceae vorteilhaft sein, zusätzlich zur Verstärkung des tig-Gens, ein oder mehrere Enzyme des bekannten Tryptophan-Biosyntheseweges oder Enzyme der Serin-Biosynthese oder Enzyme für die Produktion von 3-deoxy-D-arabino-heptulosonat-7-Phosphat (DHAP) und Chorismat zu verstärken. Die Verwendung endogener Gene wird im Allgemeinen bevorzugt.

Durch die Maßnahmen der Überexpression wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (parent strain) wird der Mikroorganismus verstanden, an dem die erfindungsgemäße Überexpression durchgeführt wird.

So können beispielsweise für die Produktion von L-Threonin gleichzeitig eines oder mehrere der Gene, ausgewählt aus der Gruppe
- mindestens ein Gen des für die Aspartatkinase, die Homoserin-Dehydrogenase, die Homoserinkinase und die Threoninsynthase kodierenden thrABC-Operons (US-A-4,278,765),
- das für die Pyruvat-Carboxylase kodierende pyc-Gen von Corynebacterium glutamicum (WO 99/18228),
- das für die Phosphoenolpyruvat-Synthase kodierende pps-Gen (Molecular and General Genetics 231(2): 332-336 (1992); WO 97/08333),
- das für die Phosphoenolpyruvat-Carboxylase kodierende ppc-Gen (WO 02/064808),
- die für die Untereinheiten der Pyridin-Transhydrogenase kodierenden Gene pntA und pntB (European Journal of Biochemistry 158: 647-653 (1986); WO 95/11985),
- das für das Threoninresistenz vermittelnde Protein kodierende Gen rhtC (EP-A-1 013 765),
- das für das Threonin-Export-Carrier-Protein kodierende thrE-Gen von Corynebacterium glutamicum (WO 01/92545),
- das für die Glutamat-Dehydrogenase kodierende gdhA-Gen (Nucleic Acids Research 11: 5257-5266 (1983); Gene 23: 199-209 (1983); DE19907347),
- das für die Phosphohistidin-Protein-Hexose-Phosphotransferase des Phosphotransferase-Systems PTS kodierende ptsH-Gen des ptsHIcrr-Operons (WO 03/004674),
- das für das Enzym I des Phosphotransferase-Systems PTS kodierende ptsI-Gen des ptsHIcrr-Operons (WO 03/004674),
- das für die Glucose-spezifische IIA Komponente des Phosphotransferase-Systems PTS kodierende crr-Gen des ptsHIcrr-Operons (WO 03/004674),
- das für die Glucose-spezifische IIBC Komponente kodierende ptsG-Gen (WO 03/004670),
- das für die Cystein-Synthase A kodierende cysK-Gen (WO 03/006666),
- das für den Regulator des cys-Regulons kodierende cysB-Gen (WO 03/006666),
- das für das Flavoprotein der NADPH-Sulfit-Reduktase kodierende cysJ-Gen des cysJIH-Operons (WO 03/006666),
- das für das Hämoprotein der NADPH-Sulfit-Reduktase kodierende cysI-Gen des cysJIH-Operons (WO 03/006666),
- das für die Adenylylsulfat-Reduktase kodierende cysH-Gen des cysJIH-Operons (WO 03/006666),
- das für die Decarboxylase Untereinheit der 2-Ketoglutarat Dehydrogenase kodierende sucA-Gen des sucABCD-Operons (WO 03/008614),
- das für die Dihydrolipoyltranssuccinase E2 Untereinheit der 2-Ketoglutarat Dehydrogenase kodierende sucB-Gen des sucABCD-Operons (WO 03/008614),
- das für die β-Untereinheit der Succinyl-CoA Synthetase kodierende sucC-Gen des suc ABCD-Operons (WO 03/008615),
- das für die α-Untereinheit der Succinyl-CoA Synthetase kodierende sucD-Gen des sucABCD-Operons (WO 03/008615),
- das Genprodukt des offenen Leserahmens (ORF) yjcG von Escherichia coli (Accession Number NC000913 (Region 4281276-4282925) des National Center for Biotechnology Information (NCBI, Bethesda, MD, USA),
- das Genprodukt des offenen Leserahmens (ORF) yibD von Escherichia coli (Accession Number NC000913 (Region 3787070-3788104) des National Center for Biotechnology Information (NCBI, Bethesda, MD, USA)),
- das Genprodukt des offenen Leserahmens (ORF) yaaU von Escherichia coli (Accession Number NC000913 (Region 45807-47138) des National Center for Biotechnology Information (NCBI, Bethesda, MD, USA), der auch unter der Bezeichnung yaaU-ORF bekannt ist,
- das für das Protein Export Chaperon SecB des Sec Protein Sekretions-Komplexes kodierende secB-Gen (Accession Number U00096.2 (Region 3781684-3782151 (komplementär) des National Center for Biotechnology Information (NCBI, Bethesda, MD, USA)),
- das für das Chaperon GroES, Regulator des Hsp60, kodierende mopB-Gen (WO 03/004669),
- das für das Chaperon Hsp70 (DnaK) kodierende dnaK-Gen (Accession Number U00096.2 (Region 12163-14079))
- das für das Chaperon DnaJ, Partner-Chaperon von DnaK, kodierende dnaJ-Gen (Accession Number U00096.2 (Region 14168-15298))
- das für das Chaperon Hsp60 (GroEL) kodierende mopA-Gen (Accession Number U00096.2 (Region 4369048-4370694)),
- das für das essentielle GTP-bindende Zellteilungsprotein FtsZ kodierende ftsZ-Gen (Accession Number U00096.2 (Region 105305-106456)),
- das für das Chaperon Hsp90 (HtpG) kodierende htpG-Gen (Accession Number U00096.2 (Region 494344-496218))
   überexprimiert werden.

Desweiteren können beispielsweise für die Produktion von L-Tryptophan, gleichzeitig eines oder mehrere der Gene, ausgewählt aus der Gruppe
- mindestens ein Gen des für die Anthranilat-Synthase, die Anthranilat-Phosphoribosyltransferase, die Phosphoribosylanthranilat-Isomerase, die Indol-3-Glyzerinphosphat-Synthase und die Tryptophan-Synthase kodierenden Tryptophan-Operons (Applied and Environmental Microbiology 38(2): 181-190 (1979)),
- das für die 3-Phosphoglycerat-Dehydrogenase kodierende serA-Gen (WO87/01130)
- das für die Phosphoserinphosphatase kodierende serB-Gen (WO87/01130)
- das für die Phosphoserinaminotransferase kodierende serC-Gen (WO87/01130)
- das für die L-Tyrosin sensitive DHAP-Synthase kodierende aroF-Gen (WO87/01130; EP1270721)
- das für die L-Phenylalanin sensitive DHAP-Synthase kodierende aroG-Gen (WO87/01130; EP1270721)
- das für die L-Tryptophan sensitive DHAP-Synthase kodierende aroH-Gen (WO87/01130; EP1270721)
- das für die Phosphoenolpyruvat-Synthase kodierende pps-Gen (WO96/08567)
- das für die Phosphoenolpyruvat-Carboxykinase kodierende pckA-Gen (WO2004/090125),
- das für die Transketolase A kodierende tktA-Gen (US5, 168, 056),
- das für die Transketolase B kodierende tktB-Gen (US5, 168, 056),
- das Genprodukt des offenen Leserahmens (ORF) yddG von Escherichia coli (Accession Number NC000913 (Region 1544312-1545193) des National Center for Biotechnology Information (NCBI, Bethesda, MD, USA)),
- das für das Protein Export Chaperon SecB des Sec Protein Sekretions-Komplexes kodierende secB-Gen (Accession Number U00096.2 (Region 3781684-3782151 (komplementär) des National Center for Biotechnology Information (NCBI, Bethesda, MD, USA)),
- das für das Chaperon GroES, Regulator des Hsp60, kodierende mopB-Gen (WO 03/004669),
- das für das Chaperon Hsp70 (DnaK) kodierende dnaK-Gen (Accession Number U00096.2 (Region 12163-14079))
- das für das Chaperon DnaJ, Partner-Chaperon von DnaK, kodierende dnaJ-Gen (Accession Number U00096.2 (Region 14168-15298))
- das für das Chaperon Hsp60 (GroEL) kodierende mopA-Gen (Accession Number U00096.2 (Region 4369048-4370694)),
- das für das essentielle GTP-bindende Zellteilungsprotein FtsZ kodierende ftsZ-Gen (Accession Number U00096.2 (Region 105305-106456)),
- das für das Chaperon Hsp90 (HtpG) kodierende htpG-Gen (Accession Number U00096.2 (Region 494344-496218)) überexprimiert werden.

L-Threonin produzierende Mikroorganismen der Familie Enterobacteriaceae besitzen typischerweise eine "feed back" resistente bzw. desensibilisierte Aspartatkinase I/Homoserin-Dehydrogenase I. Unter "feed back" resistenter Aspartatkinase/Homoserin-Dehydrogenase versteht man Aspartatkinase/Homoserin-Dehydrogenase Enzyme (kodiert durch thrA, EC:2.7.2.4/EC:1.1.1.3), die im Vergleich zur Wildform eine geringere Empfindlichkeit gegenüber der Hemmung durch Threonin oder Mischungen von Threonin und dem Threonin-Analogon α-Amino-β-Hydroxyvaleriansäure (AHV) oder AHV allein aufweisen. Die für diese desensibilisierten Enzyme kodierenden Gene bzw. Allele werden auch als thrA^{FBR}-Allele bezeichnet. Im Stand der Technik sind thrA^{FBR}-Allele beschrieben, die für Aspartatkinase/Homoserin-Dehydrogenase Varianten kodieren, welche Aminosäureaustausche im Vergleich zum Wildtypprotein besitzen. Die Kodierregion des Wildtyp thrA-Gens von Escherichia coli entsprechend der Zugangsnummer U00096.2 der NCBI Datenbank (Bethesda, MD, USA) ist in SEQ ID NO:7 und das von diesem Gen kodierte Polypeptid in SEQ ID NO:8 dargestellt.

Auch die Nukleotidsequenz des thrA-Gens von Serratia marcescens ist bekannt und unter der Zugangsnummer X60821 beim NCBI verfügbar. Die Kodierregion des Wildtyp thrA-Gens von Serratia marcescens ist in SEQ ID NO:9 und das von diesem Gen kodierte Polypeptid in SEQ ID NO:10 dargestellt.

Die für die Maßnahmen der Erfindung eingesetzten L-Threonin produzierenden Mikroorganismen der Familie Enterobacteriaceae verfügen bevorzugt über ein thrA-Allel, das für eine Aspartatkinase/Homoserin-Dehydrogenase Variante kodiert, welche die Aminosäuresequenz von SEQ ID NO:8 oder SEQ ID NO:10 besitzt, wobei diese eine oder mehrere der Aminosäureaustausche ausgewählt aus der Gruppe
ThrA E253K (Austausch von L-Glutaminsäure an Position 253 des kodierten Enzyms Aspartatkinase/Homoserin-Dehydrogenase gemäß SEQ ID NO:8 oder SEQ ID NO:10 gegen L-Lysin; siehe Research Disclosure 505, 537 (2006)),
ThrA G330D (Austausch von Glycin an Position 330 des kodierten Enzyms Aspartatkinase/Homoserin-Dehydrogenase gemäß SEQ ID NO:8 oder SEQ ID NO:10 gegen L-Asparaginsäure; siehe Omori et al. (Journal of Bacteriology 175(3), 785-794 (1993)),
ThrA S345F (Austausch von L-Serin an Position 345 des kodierten Enzyms Aspartatkinase/Homoserin-Dehydrogenase gemäß SEQ ID NO:8 oder SEQ ID NO:10 gegen L-Phenylalanin; siehe Lee et al., Journal of Bacteriology 185 (18) : 5442-5451 (2003)),
ThrA S352, Austausch von L-Serin an Position 352 des kodierten Enzyms Aspartatkinase/Homoserin-Dehydrogenase gemäß SEQ ID NO:8 oder SEQ ID NO:10 gegen L-Phenylalanin, L-Tyrosin, L-Asparagin, L-Alanin, L-Arginin, L-Glutamin, L-Glutaminsäure, L-Histidin, L-Leucin, L-Methionin, L-Tryptophan oder L-Valin, bevorzugt gegen L-Phenylalanin; siehe Omori et al. (Journal of Bacteriology 175(3), 785-794 (1993) und Omori et al. (Journal of Bacteriology 175(4), 959-965 (1993),
ThrA A479T (Austausch von L-Alanin an Position 479 des kodierten Enzyms Aspartatkinase/Homoserin-Dehydrogenase gemäß SEQ ID NO:8 oder SEQ ID NO:10 gegen L-Threonin; siehe Omori et al. (Journal of Bacteriology 175(3), 785-794 (1993)),
umfasst.

Bevorzugt wird eines der thrA^{FBk}-Allele thrA E253K (Austausch von L-Glutaminsäure an Position 253 des kodierten Enzyms Aspartatkinase/Homoserin-Dehydrogenase gegen L-Lysin)oder S345F (Austausch von L-Serin an Position 345 des kodierten Enzyms Aspartatkinase/Homoserin-Dehydrogenase gegen L-Phenylalanin) gemäß SEQ ID NO:8.

Die hier beschriebenen thrA^{FBR}-Allele, die für ein Aspartatkinase/Homoserin-Dehydrogenase Enzym kodieren, können mit den oben beschriebenen Maßnahmen überexprimiert werden.

Weiterhin kann es für die Produktion von L-Threonin und L-Tryptophan vorteilhaft sein, zusätzlich zur Verstärkung des tig-Gens, unerwünschte Nebenreaktionen auszuschalten (Nakayama: "Breeding of Amino Acid Producing Microorganisms", in: Overproduction of Microbial Products, Krumphanzl, Sikyta, Vanek (eds.), Academic Press, London, UK, 1982).

So können beispielsweise für die Produktion von L-Threonin gleichzeitig eines oder mehrere der Gene, ausgewählt aus der Gruppe
- das für die Threonin-Dehydrogenase kodierende tdh-Gen (Journal of Bacteriology 169: 4716-4721 (1987)),
- das für die Malat-Dehydrogenase (E.C. 1.1.1.37) kodierende mdh-Gen (Archives in Microbiology 149: 36-42 (1987)),
- das für das Enzym Phosphoenolpyruvat-Carboxykinase kodierende pckA-Gen (WO 02/29080),
- das für die Pyruvat-Oxidase kodierende poxB-Gen (WO 02/36797),
- das für den DgsA-Regulator des Phosphotransferase-Systems kodierende dgsA-Gen (WO 02/081721), das auch unter der Bezeichnung mlc-Gen bekannt ist,
- das für den Fructose-Repressor kodierende fruR-Gen (WO 02/081698), das auch unter der Bezeichnung cra-Gen bekannt ist,
- das für den Sigma³⁸-Faktor kodierende rpoS-Gen (WO 01/05939), das auch unter der Bezeichnung katF-Gen bekannt ist,
- das für die Aspartat Ammonium-Lyase kodierende aspA-Gen (WO 03/008603),
- das für das Outer Membrane Porin F kodierende ompF-Gen (Accession Number U00096.2 (Region 985117-986205 (komplementär))
ausgeschaltet oder die Expression verringert werden. Diese Massnahmen werden gegebenenfalls zusätzlich zu bzw. in geeigneter Kombination mit den angegebenen Massnahmen der Überexpression von Genen zur Erhöhung der Threonin-Produktion durchgeführt.

Weiterhin kann es für die Produktion von L-Tryptophan vorteilhaft sein, zusätzlich zur Überexpression des tig-Gens, eines oder mehrere der Gene ausgewählt aus der Gruppe
- das für die Tryptophanase kodierende tnaA-Gen (US4, 371, 614),
- das für den Repressor des trp-Operons kodierende trpR-Gen (US4, 371, 614)
- das für die Chorismat-Mutase T und Prephenat-Dehydrogenase kodierende tyrA-Gen (US4,371,614),
- das für die Chorismat-Mutase P und Prephenat-Dehydrogenase kodierende pheA-Gen (US4,371,614),das für das Tryptophan spezifische Transportprotein kodierende mtr-Gen (US5,756,345),
- das für die Tryptophan-Permease kodierende tnaB-Gen (US5,756,345),
- das für den Transporter für aromatische Aminosäuren kodierende aroP-Gen (US5,756,345),
- das für die L-Serin Deaminase kodierende sdaA-Gen (EP0149539) ,
- das für die Glukose-6-Phosphat-Isomerase kodierende pgi-Gen (WO87/01130),
- das für die Tyrosin-Aminotransferase kodierende tyrB-Gen (WO87/01130),
- das für das Outer Membrane Porin F kodierende ompF-Gen (Accession Number U00096.2 (Region 985117-986205 (komplementär))
auszuschalten oder die Expression zu verringern. Diese Massnahmen werden gegebenenfalls zusätzlich zu bzw. in geeigneter Kombination mit den angegebenen Massnahmen der Überexpression von Genen zur Erhöhung der Tryptophan-Produktion durchgeführt.

Durch die Maßnahmen der Verringerung der Expression wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im für das entsprechende Enzym bzw. Protein nicht rekombinanten Mikroorganismus oder Elternstamm, herabgesenkt. Als nicht rekombinanter Mikroorganismus oder Elternstamm (parent strain) wird der Mikroorganismus verstanden, an dem die erfindungsgemäße Verringerung der Expression oder Ausschaltung durchgeführt wird.

Die Verringerung der Genexpression kann durch geeignete Kulturführung, durch genetische Veränderung (Mutation) der Signalstrukturen der Genexpression oder auch durch Antisense-RNA Technik erfolgen. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann unter anderem beispielsweise bei Jensen und Hammer (Biotechnology and Bioengineering 58: 191-195 (1998)), bei Carrier und Keasling (Biotechnology Progress 15: 58-64 (1999)), Franch und Gerdes (Current Opinion in Microbiology 3: 159-164 (2000)), Kawano et al. (Nucleic Acids Research 33(19), 6268-6276 (2005)) und in bekannten Lehrbüchern der Genetik und Molekularbiologie wie beispielsweise dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Die Leistung der isolierten Bakterien bzw. des Fermentationsprozesses unter Verwendung derselben bezüglich eines oder mehrerer der Parameter ausgewählt aus der Gruppe der Produkt-Konzentration (Produkt pro Volumen), der Produkt-Ausbeute (gebildetes Produkt pro verbrauchter Kohlenstoff-Quelle) und der Produkt-Bildung (gebildetes Produkt pro Volumen und Zeit) oder auch anderer Prozess-Parameter und Kombinationen davon, wird um mindestens 0,5%, mindestens 1%, mindestens 1,5% oder mindestens 2% bezogen auf den nicht rekombinanten Mikroorganismus oder Elternstamm bzw. den Fermentationsprozess unter Verwendung desselben erhöht.

Erfindungsgemäß werden die hergestellten Mikroorganismen im batch - Verfahren (Satzkultivierung), im fed batch (Zulaufverfahren), im repeated fed batch-Verfahren (repetitives Zulaufverfahren) oder in einem kontinuierlichen Verfahren (DE102004028859.3 oder US5,763,230) kultiviert. Zusammenfassungen über derartige Verfahren sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Bei einem batch-Verfahren werden, mit wenigen Ausnahmen, wie beispielsweise Sauerstoff und pH-Korrekturmittel, sämtliche Einsatzstoffe in Form eines Ansatzes vorgelegt und der Mikrorganismus in dem erhaltenen Medium kultiviert.

Bei einem fed batch-Verfahren wird der Mikrorganismus zunächst mittels eines batch-Verfahrens kultiviert (batch-Phase). Im Anschluss daran wird der Kultur ein für die Herstellung des Produktes wesentlicher Einsatzstoff, ggf. auch mehrere Einsatzstoffe, kontinuierlich oder diskontinuierlich hinzugefügt (Zulaufphase, feed-Phase). Im Falle der Herstellung von L-Aminosäuren handelt es sich hierbei um eine Kohlenstoffquelle.

Bei einem repeated fed batch-Verfahren handelt es sich um ein fed batch-Verfahren, bei dem nach Abschluss der Fermentation ein Teil der erhaltenen Fermentationsbrühe als Inokulum zum Start eines erneuten repeated fed batch-Verfahrens dient. Dieser Zyklus kann ggf. mehrfach wiederholt werden. Repeated fed batch-Verfahren sind beispielsweise in der WO 02/18543 und WO 05/014843 beschrieben.

Bei einem kontinuierlichen Verfahren werden im Anschluß an ein batch- oder fed batch-Verfahren ein oder mehrere ggf. sämtliche Einsatzstoffe zu der Kultur kontinuierlich hinzugefügt und gleichzeitig Fermentationsbrühe entnommen. Kontinuierliche Verfahren sind beispielsweise in den Patentschriften US 5,763,230, WO 05/014840, WO 05/014841 und WO 05/014842 beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium, Fermentationsmedium und Nährmedium bzw. Medium sind gegenseitig austauschbar.

Im Allgemeinen enthält ein Kulturmedium unter anderem eine oder mehrere Kohlenstoffquelle(n), Stickstoffquelle(n) und Phosphorquelle(n).

Als Kohlenstoffquelle können Zucker und Kohlehydrate wie z.B. Glucose, Saccharose, Laktose, Fructose, Maltose, Melasse, Stärke und gegebenenfalls Cellulose, Öle und Fette wie z.B. Sojaöl, Sonnenblumenöl, Erdnussöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muss weiterhin Salze von Metallen enthalten, wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Die Fermentation wird im Allgemeinen bei einem pH-Wert von 5,5 bis 9,0, insbesondere 6,0 bis 8,0, durchgeführt. Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 25°C bis 45°C und vorzugsweise bei 30°C bis 40°C. Durch die Tätigkeit der Mikroorganismen kommt es zu einer Anreicherung bzw. Akkumulation der L-Aminosäure in der Fermentations- bzw. Kulturbrühe. Die Kultur wird solange fortgesetzt, bis sich ein Maximum an der gewünschten L-Aminosäure gebildet hat. Dieses Ziel wird normalerweise innerhalb von 10 Stunden bis 160 Stunden erreicht. Bei kontinuierlichen Verfahren sind längere Kultivierungszeiten möglich.

Unter einer Fermentationsbrühe bzw. Kulturbrühe versteht man ein Fermentationsmedium, in dem ein Mikroorganismus für eine gewisse Zeit und bei einer gewissen Temperatur kultiviert wurde.

Bei Abschluss der Fermentation enthält die entstandene Fermentationsbrühe dementsprechend a) die infolge der Vermehrung der Zellen des Mikroorganismus entstandene Biomasse (Zellmasse) des Mikroorganismus, b) die im Laufe der Fermentation gebildete L-Aminosäure, c) die im Laufe der Fermentation gebildeten organischen Nebenprodukte und d) die durch die Fermentation nicht verbrauchten Bestandteile des/ der eingesetzten Fermentationsmediums/ Fermentationsmedien bzw. der Einsatzstoffe wie beispielsweise Vitamine wie Thiamin oder Salze wie Magnesiumsulfat.

Die hergestellte Kultur- bzw. Fermentationsbrühe kann anschließend gesammelt und die gewünschte L-Aminosäure bzw. das L-Aminosäure-haltige Produkt gewonnen oder isoliert werden.

In einer Verfahrensvariante wird die Fermentationsbrühe gegebenenfalls konzentriert und anschließend die L-Aminosäure gereinigt bzw. in reiner oder nahezu reiner Form isoliert. Typische Methoden zur Reinigung von L-Aminosäuren sind die Ionenaustauschchromatographie, die Kristallisation, Extraktionsverfahren und die Behandlung mit Aktivkohle. Hierdurch erhält man weitgehend reine L-Aminosäuren mit einem Gehalt von ≥ 90 Gew.-%, ≥ 95 Gew.-%, ≥ 96 Gew.-%, ≥ 97 Gew.-% ≥ 98 Gew.-% oder ≥ 99 Gew.-%.

Es ist ebenfalls möglich in einer anderen Verfahrensvariante aus der hergestellten Fermentationsbrühe ein Produkt herzustellen, indem man die in der Fermentationsbrühe enthaltene Biomasse des Bakteriums vollständig (100%) oder nahezu vollständig d.h. mehr als oder größer als (>) 90%, >95%, >97%, >99% entfernt und die übrigen Bestandteile der Fermentationsbrühe weitgehend d.h. zu 30% - 100%, 40% - 100%, 50% - 100%, 60% - 100%, 70% - 100%, 80% - 100%, oder 90% - 100%, bevorzugt größer gleich (≥) 50%, ≥60%, ≥70%, ≥80%, ≥90% oder ≥95% oder auch vollständig (100%) im Produkt belässt.

Zur Entfernung oder Abtrennung der Biomasse werden Separationsmethoden wie beispielsweise Zentrifugation, Filtration, Dekantieren, Flockung oder eine Kombination hieraus eingesetzt.

Die erhaltene Brühe wird anschließend mit bekannten Methoden wie beispielsweise mit Hilfe eines Rotationsverdampfers, Dünnschichtverdampfers, Fallfilmverdampfers, durch Umkehrosmose, durch Nanofiltration oder einer Kombination hieraus eingedickt beziehungsweise konzentriert.

Diese aufkonzentrierte Brühe wird anschließend durch Methoden der Gefriertrocknung, der Sprühtrocknung, der Sprühgranulation oder durch anderweitige Verfahren zu einem vorzugsweise rieselfähigen, feinteiligen Pulver aufgearbeitet. Dieses rieselfähige, feinteilige Pulver kann dann wiederum durch geeignete Kompaktier- oder GranulierVerfahren in ein grobkörniges, gut rieselfähiges, lagerbares und weitgehend staubfreies Produkt überführt werden. Das Wasser wird hierbei insgesamt zu mehr als 90% entfernt, sodass der Wassergehalt im Produkt kleiner als 10 Gew.-%, kleiner als Gew.-5%, kleiner als Gew.-4% oder kleiner 3 Gew.-% beträgt.

Die Analyse von L-Aminosäuren kann durch Anionenaustauschchromatographie mit anschließender Ninhydrin Derivatisierung erfolgen, so wie bei Spackman et al. (Analytical Chemistry 30: 1190-1206 (1958)) beschrieben, oder sie kann durch reversed phase HPLC erfolgen, so wie bei Lindroth et al. (Analytical Chemistry 51: 1167-1174 (1979)) beschrieben.

Die vorliegende Erfindung wird im Folgenden anhand von Ausführungsbeispielen näher erläutert.

Verwendete Minimal- (M9) und Vollmedien (LB) für Escherichia coli sind von J.H. Miller (A Short Course in Bacterial Genetics (1992), Cold Spring Harbor Laboratory Press) beschrieben. Die Isolierung von Plasmid-DNA aus Escherichia coli sowie alle Techniken zur Restriktion, Ligation, Klenow- und alkalische Phosphatasebehandlung werden nach Sambrook et al. (Molecular Cloning - A Laboratory Manual (1989) Cold Spring Harbor Laboratory Press) durchgeführt. Die Transformation von Escherichia coli wird, wenn nicht anders beschrieben, nach Chung et al. (Proceedings of the National Academy of Sciences of the United States of America 86: 2172-2175 (1989)) durchgeführt.

Die Bebrütungstemperatur bei der Herstellung von Stämmen und Transformanten ist 37°C.

### Beispiel 1

### Konstruktion des Expressionsplasmides pTrc99Atig

Das tig-Gen aus E. coli K12 wird unter Anwendung der Polymerase-Kettenreaktion (PCR) sowie synthetischen Oligonukleotiden amplifiziert. Ausgehend von der Nukleotidsequenz des tig-Gens in E. coli K12 MG1655 (Accession Number U00096 (Region: (454357-455655)), Blattner et al. (Science 277: 1453-1474 (1997)) werden PCR-Primer synthetisiert (MWG Biotech, Ebersberg, Deutschland). Die Sequenzen der Primer werden so modifiziert, dass Erkennungsstellen für Restriktionsenzyme entstehen. Für den tig-1-Primer wird die Erkennungssequenz für XbaI und für den tig-2-Primer die Erkennungssequenz für HindIII gewählt, die in der unten dargestellten Nukleotidabfolge durch Unterstreichen markiert sind:
tig-1:
   5' - GCTCTAGA AAGAGTTGAC CGAGCACTGT- 3' (SEQ ID No. 11)
tig-2:
   5' - GCAAGCTT ATTACGCCTG CTGGTTCATC- 3' (SEQ ID No. 12)

Die für die PCR eingesetzte chromosomale E. coli K12 MG1655 DNA wird nach Herstellerangaben mit "Qiagen Genomic-tips 100/G" (QIAGEN, Hilden, Deutschland) isoliert. Ein 1350 bp großes DNA-Fragment kann mit den spezifischen Primern unter Standard-PCR-Bedingungen (Innis et al. (1990) PCR Protocols. A Guide to Methods and Applications, Academic Press) mit der Vent-DNA-Polymerase (New England Biolaps GmbH, Frankfurt, Deutschland) amplifiziert werden (SEQ ID No. 13).

Das amplifizierte tig-Fragment wird mit dem Vektor pCR-Blunt II-TOPO (Zero TOPO TA Cloning Kit, Invitrogen, Groningen, Niederlande) den Herstellerangaben entsprechend ligiert und in den E. coli Stamm TOP10 transformiert. Die Selektion Plasmid tragender Zellen erfolgt auf LB Agar, der mit 50 µg/ml Kanamycin versetzt ist. Nach der Plasmid DNA Isolierung wird der Vektor mit dem Enzym EcoRI gespalten und nach Überprüfung der Spaltung im 0,8%igen Agarosegel mit pCRBlunttig bezeichnet.

Die Nukleotidsequenz des amplifizierten DNA-Fragmentes bzw. PCR-Produktes wird nach der Dideoxy-Kettenabbruch-Methode von Sanger et al. (Proceedings of the National Academy of Sciences U.S.A., 74:5463-5467 (1977)) mit dem "ABI Prism 377" Sequenziergerät der Firma PE Applied Biosystems (Weiterstadt, Deutschland) überprüft. Die Sequenz des PCR-Produktes entspricht den Positionen 1-1299 der in der SEQ ID No. 1 dargestellten Sequenz. Die Aminosäuresequenz des dazugehörigen Tig-Proteins ist in der SEQ ID No. 14 dargestellt.

Anschließend wird der Vektor pCRBlunttig mit den Enzymen HindIII und XbaI gespalten und das ca. 1350bp tig-Fragment nach Auftrennung im 0,8%igem Agarosegel aus dem Gel isoliert (QIAquick Gel Extraction Kit, QIAGEN, Hilden, Deutschland) und mit dem Vektor pTrc99A (Pharmacia Biotech, Uppsala, Schweden), der mit den Enzymen HindIII und XbaI verdaut worden ist, ligiert. Der E. coli Stamm DH5α (Grant et al.; Proceedings of the National Academy of Sciences USA, 87 (1990) 4645-4649) wird mit dem Ligationsansatz transformiert und Plasmid tragende Zellen auf LB Agar, der mit 50 µg/ml Ampicillin versetzt ist, selektioniert.

Die erfolgreiche Klonierung kann nach der Plasmid DNA Isolierung durch die Kontrollspaltung mit den Enzymen EcoRI bzw. PstI nachgewiesen werden.

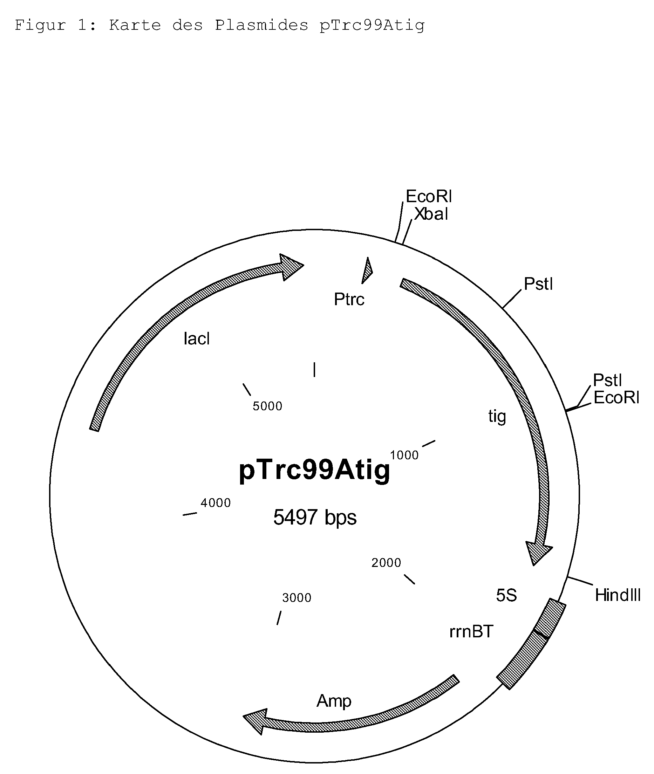

Das Plasmid wird als pTrc99Atig (Figur 1) bezeichnet.

### Beispiel 2

Herstellung von L-Threonin mit dem Stamm MG442/pTrc99Atig Der L-Threonin produzierende E. coli Stamm MG442 ist in der Patentschrift US-A- 4,278,765 beschrieben und bei der Russischen Nationalsammlung für industrielle Mikroorganismen (VKPM, Moskau, Russland) als CMIM B-1628 hinterlegt.

Der Stamm MG442 wird mit dem in Beispiel 1 beschriebenen Expressionsplasmid pTrc99Atig und mit dem Vektor pTrc99A transformiert und auf LB Agar mit 50 µg/ml Ampicillin Plasmid tragende Zellen selektioniert. Auf diese Weise entstehen die Stämme MG442/pTrc99Atig und MG442/pTrc99A. Ausgewählte Einzelkolonien werden anschließend auf Minimalmedium mit der folgenden Zusammensetzung: 3,5 g/l Na₂HPO₄*2H₂O, 1,5 g/l KH₂PO₄, 1 g/l NH₄Cl, 0,1 g/l MgSO₄*7H₂O, 2 g/l Glucose, 20 g/l Agar, 50 mg/l Ampicillin, weiter vermehrt. Die Bildung von L-Threonin wird in batch Kulturen von 10 ml, die in 100 ml Erlenmeierkolben enthalten sind, überprüft. Dazu wird 10 ml Vorkulturmedium der folgenden Zusammensetzung: 2 g/l Hefeextrakt, 10 g/l (NH₄)₂SO₄, 1 g/l KH₂PO₄, 0,5 g/l MgSO₄*7H₂O, 15 g/l CaCO₃, 20 g/l Glucose, 50 mg/l Ampicillin, beimpft und für 16 Stunden bei 37°C und 180 rpm auf einem ESR Inkubator der Firma Kühner AG (Birsfelden, Schweiz) inkubiert. Je 250 µl dieser Vorkultur werden in 10 ml Produktionsmedium (25 g/l (NH₄)₂SO₄, 2 g/l KH₂PO₄, 1 g/l MgSO₄*7H₂O, 0,03 g/l FeSO₄*7H₂O, 0,018 g/l MnSO₄*1H₂O, 30 g/l CaCO₃, 20 g/l Glucose, 50 mg/l Ampicillin) überimpft und für 48 Stunden bei 37°C inkubiert. Nach der Inkubation wird die optische Dichte (OD) der Kultursuspension mit einem LP2W-Photometer der Firma Dr. Lange (Düsseldorf, Deutschland) bei einer Messwellenlänge von 660 nm bestimmt.

Anschließend wird die Konzentration an gebildetem L-Threonin im steril filtrierten Kulturüberstand mit einem Aminosäureanalysator der Firma Eppendorf-BioTronik (Hamburg, Deutschland) durch Ionenaustauschchromatographie und Nachsäulenreaktion mit Ninhydrindetektion bestimmt.

In Tabelle 1 ist das Ergebnis des Versuches dargestellt.

**Tabelle 1**

| Stamm | OD (660 nm) | L-Threonin g/l |
|---|---|---|
| MG442/pTrc99A | 3, 0 | 1, 3 |
| MG442/pTrc99Atig | 5,1 | 1, 9 |

Kurze Beschreibung der Figuren:
Figur 1: Karte des das tig-Gen enthaltenden Plasmides pTrc99Atig
   Längenangaben sind als ca.-Angaben aufzufassen. Die verwendeten Abkürzungen und Bezeichnungen haben folgende Bedeutung:
   - Amp: Gen, welches für die Ampicillinresistenz kodiert
   - lacI: Gen für das Repressorprotein des trc-Promotors
   - Ptrc: trc-Promotorregion, IPTG-induzierbar
   - tig: Kodierregion des tig-Gens
   - 5S: 5S rRNA-Region
   - rrnBT: rRNA-Terminator-Region

Die Abkürzungen für die Restriktionsenzyme haben folgende Bedeutung:
- XbaI: Restriktionsendonuklease aus Xanthomonas badrii
- HindIII: Restriktionsendonuklease aus Haemophilus influenzae
- EcoRI: Restriktionsendonuklease aus Escherichia coli
- PstI: Restriktionsendonuklease aus Providencia stuartii

## Patentansprüche

1. Rekombinante L-Threonin oder L-Tryptophan produzierende Mikroorganismen der Familie Enterobacteriaceae, die ein überexprimiertes tig-Gen enthalten, welches für den Triggerfaktor TF kodiert, wobei die für die Maßnahmen der Überexpression des tig-Gens eingesetzten Elternstämme bereits die Fähigkeit besitzen, mindestens 0,25 g/l L-Threonin oder L-Tryptophan in maximal 120 Stunden in der Zelle und/oder im Nährmedium beziehungsweise der Fermentationsbrühe anzureichern, wobei
a) die Mikroorganismen durch Transformation, Transduktion, Konjugation oder eine Kombination dieser Methoden hergestellt werden, mit einem Vektor, der das tig-Gen, ein Allel dieses Gens oder Teile davon, die die Aktivität des Triggerfaktors TF aufweisen, und gegebenenfalls einen Promotor enthält; und/oder
b) in den Mikroorganismen die Kopienzahl des tig-Gens oder der Allele im Vergleich zu den für das tig-Gen nicht rekombinanten Mikroorganismen um mindestens 1 erhöht vorliegt; und/oder
c) man zur Erzielung der Verstärkung die Promotor- und Regulationsregion oder die Ribosomenbindungsstelle stromaufwärts des tig-Gens mutiert oder Expressionskassetten oder Promotoren stromaufwärts des tig-Gens einbaut.

2. Mikroorganismen gemäss Anspruch 1, in denen ein dem tig-Gen entsprechendes Polynukleotid überexprimiert wird, das für ein Polypeptid kodiert, dessen Aminosäuresequenz zu mindestens 90% identisch ist mit einer Aminosäuresequenz, ausgewählt aus der Gruppe SEQ ID No. 2, SEQ ID No. 4 und SEQ ID No. 6 .

3. Mikroorganismen gemäss Anspruch 2, **dadurch gekennzeichnet, dass** sie ein dem tig-Gen entsprechendes isoliertes Polynukleotid enthalten ausgewählt aus der Gruppe:
a) Polynukleotid mit einer Nukleotidsequenz, ausgewählt aus SEQ ID No. 1, SEQ ID No. 3 oder SEQ ID No. 5 und den dazu komplementären Nukleotidsequenzen;
b) Polynukleotid mit einer Nukleotidsequenz, die der SEQ ID No. 1, SEQ ID No. 3 oder SEQ ID No. 5 im Rahmen der Degeneration des genetischen Codes entspricht;
c) Polynukleotidsequenz mit einer Sequenz, die mit der zur Sequenz SEQ ID No. 1, SEQ ID No. 3 oder SEQ ID No. 5 komplementären Sequenz unter stringenten Bedingungen hybridisiert, wobei die stringenten Bedingungen durch einen Waschschritt erreicht werden, in dem sich die Temperatur über einen Bereich von 64°C bis 68°C und die Salzkonzentration des Puffers über einen Bereich von 2xSSC bis 0,1xSSC erstrecken;
d) Polynukleotid mit einer Sequenz SEQ ID No. 1, SEQ ID No. 3 oder SEQ ID No. 5, die funktionsneutrale Sinnmutanten enthält,
wobei die Polynukleotide für den Triggerfaktor TF kodieren.

4. Mikroorganismen gemäss Anspruch 2, **dadurch gekennzeichnet, dass** das Polypeptid eine Aminosäuresequenz besitzt, die zu wenigstens 95% identisch ist mit einer der Sequenzen ausgewählt aus der Gruppe SEQ ID No. 2, SEQ ID No. 4 und SEQ ID No. 6.

5. Mikroorganismen gemäss Anspruch 2, **dadurch gekennzeichnet, dass** das Polypeptid die Aminosäuresequenz aufweist, die 100% identisch ist mit einer der Sequenzen, ausgewählt aus der Gruppe SEQ ID No. 2 oder SEQ ID No. 4.

6. Mikroorganismen gemäss Anspruch 1, in denen die Kopienzahl des tig-Gens oder der Allele um höchstens 8, bevorzugt höchstens 4 erhöht vorliegt.

7. Mikroorganismen gemäss Anspruch 6, **dadurch gekennzeichnet, dass** die Erhöhung der Kopienzahl des tig-Gens um mindestens 1 durch Integration des Gens oder der Allele in das Chromosom der Mikroorganismen bewirkt wird.

8. Mikroorganismen gemäss Anspruch 6, **dadurch gekennzeichnet, dass** die Erhöhung der Kopienzahl des tig-Gens um mindestens 1 durch einen extrachromosomal replizierenden Vektor erzielt wird.

9. Mikroorganismen gemäss den Ansprüchen 1 bis 8, **dadurch gekennzeichnet, dass** durch die Verstärkung des tig-Gens die Konzentration oder Aktivität des tig-Genproduktes (Proteins) um mindestens 10% erhöht sind, bezogen auf die Aktivität oder Konzentration des Genproduktes im für das tig-Gen nicht rekombinanten Mikroorganismus oder Elternstamm.

10. Mikroorganismen gemäss den Ansprüchen 1 bis 9, **dadurch gekennzeichnet, dass** die Mikroorganismen aus den Gattungen Escherichia, Erwinia, Providencia und Serratia ausgewählt sind.

11. Mikroorganismen gemäss den Ansprüchen 1 bis 10, **dadurch gekennzeichnet, dass** sie L-Threonin produzieren.

12. Verfahren zur Herstellung der L-Aminosäuren L-Threonin oder L-Tryptophan durch Fermentation von rekombinanten Mikroorganismen der Familie Enterobacteriaceae gemäß den Ansprüchen 1 bis 11, **dadurch gekennzeichnet, dass** man
a) die L-Threonin oder L-Tryptophan produzierenden Mikroorganismen, die ein überexprimiertes tig-Gen enthalten, welches für den Triggerfaktor TF kodiert, in einem Medium kultiviert unter Bedingungen, bei denen die gewünschte L-Aminosäure L-Threonin oder L-Tryptophan im Medium oder in den Zellen angereichert wird, und
b) die gewünschte L-Aminosäure L-Threonin oder L-Tryptophan isoliert, wobei ggf. weitere Bestandteile der Fermentationsbrühe und/oder die Biomasse in ihrer Gesamtheit oder Anteilen (≥ 0 bis 100%) im isolierten Produkt verbleiben oder vollständig entfernt werden.

13. Verfahren gemäss Anspruch 12, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) mindestens ein Gen des für die Aspartatkinase, die Homoserin-Dehydrogenase, die Homoserinkinase und die Threoninsynthase kodierenden thrABC-Operons,
b) das für die Pyruvat-Carboxylase kodierende pyc-Gen von Corynebacterium glutamicum,
c) das für die Phosphoenolpyruvat-Synthase kodierende pps-Gen,
d) das für die Phosphoenolpyruvat-Carboxylase kodierende ppc-Gen,
e) die für die Untereinheiten der Pyridin-Transhydrogenase kodierenden Gene pntA und pntB,
f) das für das Threoninresistenz vermittelnde Protein kodierende Gen rhtC,
g) das für das Threonin-Export-Carrier-Protein kodierende thrE-Gen aus Corynebacterium glutamicum,
h) das für die Glutamat-Dehydrogenase kodierende gdhA-Gen,
i) das für die Phosphohistidin-Protein-Hexose-Phosphotransferase kodierende ptsH-Gen,
j) das für das Enzym I des Phosphotransferase-Systems kodierende ptsI-Gen,
k) das für die Glucose-spezifische IIA Komponente des Phosphotransferasesystems kodierende crr-Gen,
l) das für die Glucose-spezifische IIBC Komponente kodierende ptsG-Gen,
m) das für die Cystein-Synthase A kodierende cysK-Gen,
n) das für den Regulator des cys-Regulons kodierende cysB-Gen,
o) das für das Flavoprotein der NADPH-Sulfit-Reduktase kodierende cysJ-Gen,
p) das für das Hämoprotein der NADPH-Sulfit-Reduktase kodierende cysI-Gen,
q) das für die Adenylylsulfat-Reduktase kodierende cysH-Gen,
r) das für die Decarboxylase Untereinheit der 2-Ketoglutarat Dehydrogenase kodierende sucA-Gen,
s) das für die Dihydrolipoyltranssuccinase E2 Untereinheit der 2-Ketoglutarat Dehydrogenase kodierende sucB-Gen,
t) das für die β-Untereinheit der Succinyl-CoA Synthetase kodierende sucC-Gen,
u) das für die α-Untereinheit der Succinyl-CoA Synthetase kodierende sucD-Gen,
v) das Genprodukt des offenen Leserahmens (ORF) yjcG von Escherichia coli,
w) das Genprodukt des offenen Leserahmens (ORF) yibD von Escherichia coli,
x) das Genprodukt des offenen Leserahmens (ORF) yaaU von Escherichia coli,
y) das für das Protein Export Chaperon SecB des Sec Protein Sekretions-Komplexes kodierende secB-Gen,
z) das für das Chaperon GroES, Regulator des Hsp60, kodierende mopB-Gen,
aa) das für das Chaperon Hsp70 (DnaK) kodierende dnaK-Gen,
bb) das für das Chaperon DnaJ, Partner-Chaperon von DnaK, kodierende dnaJ-Gen,
cc) das für das Chaperon Hsp60 (GroEL) kodierende mopA-Gen,
dd) das für das essentielle GTP-bindende Zellteilungsprotein FtsZ kodierende ftsZ-Gen, und
ee) das für das Chaperon Hsp90 (HtpG) kodierende htpG-Gen überexprimiert.

14. Verfahren gemäss Anspruch 12, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) mindestens ein Gen des für die Anthranilat-Synthase, die Anthranilat-Phosphoribosyltransferase, die Phosphoribosylanthranilat-Isomerase, die Indol-3-Glyzerinphosphat-Synthase und die Tryptophan-Synthase kodierenden Tryptophan-Operons,
b) das für die 3-Phosphoglycerat-Dehydrogenase kodierende serA-Gen,
c) das für die Phosphoserinphosphatase kodierende serB-Gen,
d) das für die Phosphoserinaminotransferase kodierende serC-Gen,
e) das für die L-Tyrosin sensitive DHAP-Synthase kodierende aroF-Gen,
f) das für die L-Phenylalanin sensitive DHAP-Synthase kodierende aroG-Gen,
g) das für die L-Tryptophan sensitive DHAP-Synthase kodierende aroH-Gen,
h) das für die Phosphoenolpyruvat-Synthase kodierende pps-Gen,
i) das für die Phosphoenolpyruvat-Carboxykinase kodierende pckA-Gen,
j) das für die Transketolase A kodierende tktA-Gen,
k) das für die Transketolase B kodierende tktB-Gen, und
l) das Genprodukt des offenen Leserahmens (ORF) yddG von Escherichia coli,
m) das für das Protein Export Chaperon SecB des Sec Protein Sekretions-Komplexes kodierende secB-Gen,
n) das für das Chaperon GroES, Regulator des Hsp60, kodierende mopB-Gen,
o) das für das Chaperon Hsp70 (DnaK) kodierende dnaK-Gen,
p) das für das Chaperon DnaJ, Partner-Chaperon von DnaK, kodierende dnaJ-Gen,
q) das für das Chaperon Hsp60 (GroEL) kodierende mopA-Gen,
r) das für das essentielle GTP-bindende Zellteilungsprotein FtsZ kodierende ftsZ-Gen, und
s) das für das Chaperon Hsp90 (HtpG) kodierende htpG-Gen überexprimiert.

15. Verfahren gemäss den Ansprüchen 12 und 13, **dadurch gekennzeichnet, dass** man zur Herstellung von L-Threonin Mikroorganismen fermentiert, in denen man zusätzlich gleichzeitig eines oder mehrere der Gene, ausgewählt aus der Gruppe:
a) das für die Threonin-Dehydrogenase kodierende tdh-Gen,
b) das für die Malat-Dehydrogenase kodierende mdh-Gen,
c) das für die Phosphoenolpyruvat-Carboxykinase kodierende pckA-Gen,
d) das für die Pyruvat-Oxidase kodierende poxB-Gen,
e) das für den DgsA-Regulator des Phosphotransferase-Systems kodierende dgsA-Gen,
f) das für den Fructose-Repressor kodierende fruR-Gen,
g) das für den Sigma³⁸-Faktor kodierende rpoS-Gen,
h) das für die Aspartat Ammonium-Lyase kodierende aspA-Gen, und
i) das für das Outer Membrane Porin F kodierende ompF-Gen ausschaltet oder die Expression verringert.

16. Verfahren gemäss den Ansprüchen den Ansprüchen 12 und 14, **dadurch gekennzeichnet, dass** man zur Herstellung von L-Tryptophan Mikroorganismen der Familie Enterobacteriaceae fermentiert, in denen man zusätzlich gleichzeitig eines oder mehrere der Gene, ausgewählt aus der Gruppe:
a) das für die Tryptophanase kodierende tnaA-Gen,
b) das für den Repressor des trp-Operons kodierende trpR-Gen,
c) das für die Chorismat-Mutase T und Prephenat-Dehydrogenase kodierende tyrA-Gen,
d) das für die Chorismat-Mutase P und Prephenat-Dehydrogenase kodierende pheA-Gen,
e) das für das Tryptophan spezifische Transportprotein kodierende mtr-Gen,
f) das für die Tryptophan-Permease kodierende tnaB-Gen,
g) das für den Transporter für aromatische Aminosäuren kodierende aroP-Gen,
h) das für die L-Serin Deaminase kodierende sdaA-Gen,
i) das für die Glukose-6-Phosphat-Isomerase kodierende pgi-Gen,
j) das für die Tyrosin-Aminotransferase kodierende tyrB-Gen, und
k) das für das Outer Membrane Porin F kodierende ompF-Gen
ausschaltet oder die Expression verringert.

17. Verfahren gemäss Anspruch 12 bis 16, **dadurch gekennzeichnet, dass** die Mikroorganismen in einem batch - Verfahren, fed batch - Verfahren, repeated fed batch-Verfahren oder kontinuierlichen Verfahren kultiviert werden.

18. Verfahren gemäss Anspruch 12 bis 17, **dadurch gekennzeichnet, dass** die Konzentration des isolierten L-Threonins oder L-Tryptophans bestimmt wird.

## Claims

1. Recombinant L-threonine- or L-tryptophan-producing microorganisms of the family Enterobacteriaceae, which contain an overexpressed tig gene which codes for the trigger factor TF, the parent strains used for the measures of overexpression of the tig gene already possessing the capacity to accumulate at least 0.25 g/l L-threonine or L-tryptophan in at most 120 hours in the cell and/or in the nutrient medium or fermentation broth, where
a) the microorganisms are produced by transformation, transduction, conjugation or a combination of these methods with a vector that contains the tig gene, an allele of this gene or parts thereof which have the activity of the trigger factor TF, and optionally a promoter; and/or
b) in the microorganisms the copy number of the tig gene or of the alleles has been increased by at least 1 in comparison to the microorganisms that are non-recombinant for the tig gene; and/or
c) to achieve the enhancement, the promoter and regulation region or the ribosome binding site is mutated upstream of the tig gene or expression cassettes or promoters are installed upstream of the tig gene.

2. Microorganisms according to Claim 1, in which a polynucleotide corresponding to the tig gene and which codes for a polypeptide, the amino acid sequence of which is at least 90% identical to an amino acid sequence selected from the group of SEQ ID No. 2, SEQ ID No. 4 and SEQ ID No. 6, is overexpressed.

3. Microorganisms according to Claim 2, **characterized in that** they contain an isolated polynucleotide corresponding to the tig gene, selected from the group consisting of:
a) polynucleotide having a nucleotide sequence selected from SEQ ID No. 1, SEQ ID No. 3 or SEQ ID No. 5 and the nucleotide sequences complementary thereto;
b) polynucleotide having a nucleotide sequence that corresponds to SEQ ID No. 1, SEQ ID No. 3 or SEQ ID No. 5 within the degeneracy of the genetic code;
c) polynucleotide sequence having a sequence that hybridizes under stringent conditions with the sequence complementary to the sequence SEQ ID No. 1, SEQ ID No. 3 or SEQ ID No. 5, where the stringent conditions are achieved by a washing step in which the temperature range is from 64°C to 68°C, and the range of salt concentrations of the buffer is from 2×SSC to 0.1×SSC;
d) polynucleotide having a sequence SEQ ID No. 1, SEQ ID No. 3 or SEQ ID No. 5 which contains functionally neutral sense mutations,
where the polynucleotides code for the trigger factor TF.

4. Microorganisms according to Claim 2, **characterized in that** the polypeptide has an amino acid sequence which is at least 95% identical to one of the sequences selected from the group of SEQ ID No. 2, SEQ ID No. 4 and SEQ ID No. 6.

5. Microorganisms according to Claim 2, **characterized in that** the polypeptide has the amino acid sequences which is 100% identical to one of the sequences selected from the group consisting of SEQ ID No. 2 or SEQ ID No. 4.

6. Microorganisms according to Claim 1, in which the copy number of the tig gene or of the alleles has been increased by at most 8, preferably at most 4.

7. Microorganisms according to Claim 6, **characterized in that** the increase in the copy number of the tig gene by at least 1 is brought about by integrating the gene or the alleles into the chromosome of the microorganisms.

8. Microorganisms according to Claim 6, **characterized in that** the increase in the copy number of the tig gene by at least 1 is achieved by means of a vector replicating extrachromosomally.

9. Microorganisms according to Claims 1 to 8, **characterized in that** enhancement of the tig gene increases the concentration or activity of the tig gene product (protein) by at least 10%, based on the activity or concentration of the gene product in the microorganism or parent strain that is non-recombinant for the tig gene.

10. Microorganisms according to Claims 1 to 9, **characterized in that** the microorganisms are selected from the genera Escherichia, Erwinia, Providencia and Serratia.

11. Microorganisms according to Claims 1 to 10, **characterized in that** they produce L-threonine.

12. Process for the production of the L-amino acids L-threonine or L-tryptophan by fermentation of recombinant microorganisms of the family Enterobacteriaceae according to Claims 1 to 11, **characterized in that**
a) the L-threonine- or L-tryptophan- producing microorganisms, which contain an overexpressed tig gene which codes for the trigger factor TF, are cultured in a medium under conditions in which the desired L-amino acid L-threonine or L-tryptophan is enriched in the medium or in the cells, and
b) the desired L-amino acid L-threonine or L-tryptophan is isolated, where optionally further constituents of the fermentation broth and/or the biomass remain in the isolated product in their entirety or in part (≥ 0 to 100%) or are completely removed.

13. Process according to Claim 12, **characterized in that** for the production of the L-threonine microorganisms are fermented, in which additionally at the same one or more of the genes selected from the group consisting of:
a) at least one gene of the thrABC operon coding for aspartate kinase, homoserine dehydrogenase, homoserine kinase and threonine synthase,
b) the pyc gene of Corynebacterium glutamicum coding for pyruvate carboxylase,
c) the pps gene coding for phosphoenol pyruvate synthase,
d) the ppc gene coding for phosphoenol pyruvate carboxylase,
e) the genes pntA and pntB coding for the subunits of pyridine transhydrogenase,
f) the gene rhtC coding for the threonine resistance-mediating protein,
g) the thrE gene from Corynebacterium glutamicum coding for the threonine export carrier protein,
h) the gdhA gene coding for glutamate dehydrogenase,
i) the ptsH gene coding for the phosphohistidine protein hexose phosphotransferase,
j) the ptsI gene coding for the enzyme I of the phosphotransferase system,
k) the crr gene coding for the glucose-specific IIA component of the phosphotransferase system,
l) the ptsG gene coding for the glucose-specific IIBC component,
m) the cysK gene coding for cysteine synthase A,
n) the cysB gene coding for the regulator of the cys regulon,
o) the cysJ gene coding for the flavoprotein of NADPH sulphite reductase,
p) the cysI gene coding for the haemoprotein of NADPH sulphite reductase,
q) the cysH gene coding for adenylyl sulphate reductase,
r) the sucA gene coding for the decarboxylase subunit of 2-ketoglutarate dehydrogenase,
s) the sucB gene coding for the dihydrolipoyltranssuccinase E2 subunit of 2-ketoglutarate dehydrogenase,
t) the sucC gene coding for the β-subunit of succinyl-CoA synthetase,
u) the sucD gene coding for the α-subunit of succinyl-CoA synthetase,
v) the gene product of the open reading frame (ORF) yjcG of Escherichia coli,
w) the gene product of the open reading frame (ORF) yibD of Escherichia coli,
x) the gene product of the open reading frame (ORF) yaaU of Escherichia coli,
y) the secB gene coding for the protein export chaperone SecB of the Sec protein secretion complex,
z) the mopB gene coding for the chaperone GroES, regulator of the Hsp60,
aa) the dnaK gene coding for the chaperone Hsp70 (DnaK),
bb) the dnaJ gene coding for the chaperone DnaJ, partner chaperone of DnaK,
cc) the mopA gene coding for the chaperone Hsp60 (GroEL),
dd) the ftsZ gene coding for the essential GTP-binding cell division protein FtsZ, and
ee) the htpG gene coding for the chaperone Hsp90 (HtpG).
is/are overexpressed.

14. Process according to Claim 12, **characterized in that** for the production of L-tryptophan microorganisms of the family Enterobacteriaceae are fermented, in which additionally at the same time one or more of the genes selected from the group consisting of:
a) at least one gene of the tryptophan operon coding for anthranilate synthase, anthranilate phosphoribosyl transferase, phosphoribosyl anthranilate isomerase, indole-3-glycerol phosphate synthase and tryptophan synthase,
b) the serA gene coding for 3-phosphoglycerate dehydrogenase,
c) the serB gene coding for phosphoserine phosphatase,
d) the serC gene coding for phosphoserine aminotransferase,
e) the aroF gene coding for L-tyrosine-sensitive DHAP synthase,
f) the aroG gene coding for L-phenylalanine-sensitive DHAP synthase,
g) the aroH gene coding for L-tryptophan-sensitive DHAP synthase,
h) the pps gene coding for phosphoenol pyruvate synthase,
i) the pckA gene coding for phosphoenol pyruvate carboxykinase,
j) the tktA gene coding for transketolase A,
k) the tktB gene coding for transketolase B, and
l) the gene product of the open reading frame (ORF) yddG of Escherichia coli,
m) the secB gene coding for the protein export chaperone SecB of the Sec protein secretion complex,
n) the mopB gene coding for the chaperone GroES, regulator of Hsp60,
o) the dnaK gene coding for the chaperone Hsp70 (Dnak),
p) the dnaJ gene coding for the chaperone DnaJ, partner chaperone of DnaK,
q) the mopA gene coding for the chaperone Hsp60 (GroEL),
r) the ftsZ gene coding for the essential GTP-binding cell division protein FtsZ, and
s) the htpG gene coding for the chaperone Hsp90 (HtpG)
is/are overexpressed.

15. Process according to Claims 12 and 13, **characterized in that** for the production of L-threonine microorganisms are fermented, in which additionally at the same time one or more of the genes selected from the group consisting of:
a) the tdh gene coding for threonine dehydrogenase,
b) the mdh gene coding for malate dehydrogenase,
c) the pckA gene coding for phosphoenol pyruvate carboxykinase,
d) the poxB gene coding for pyruvate oxidase,
e) the dgsA gene coding for the DgsA regulator of the phosphotransferase system,
f) the fruR gene coding for the fructose repressor,
g) the rpoS gene coding for the Sigma³⁸ factor,
h) the aspA gene coding for aspartate ammonium lyase, and
i) the ompF gene coding for the outer membrane porin F,
are switched off or the expression is decreased.

16. Process according to Claims 12 and 14, **characterized in that** for the production of L-tryptophan microorganisms of the family Enterobacteriaceae are fermented, in which additionally at the same time one or more of the genes selected from the group consisting of:
a) the tnaA gene coding for tryptophanase,
b) the trpR gene coding for the repressor of the trp operon,
c) the tyrA gene coding for chorismate mutase T and prephenate dehydrogenase,
d) the pheA gene coding for chorismate mutase P and prephenate dehydrogenase,
e) the mtr gene coding for the tryptophan-specific transport protein,
f) the tnaB gene coding for tryptophan permease,
g) the aroP gene coding for the transporter for aromatic amino acids,
h) the sdaA gene coding for L-serine deaminase,
i) the pgi gene coding for glucose-6-phosphate isomerase,
j) the tyrB gene coding for tyrosine aminotransferase, and
k) the ompF gene coding for the Outer Membrane porin F,
are switched off or the expression is decreased.

17. Process according to Claims 12 to 16, **characterized in that** the microorganisms are cultured in a batch process, fed batch process, repeated fed batch process or continuous process.

18. Process according to Claims 12 to 17, **characterized in that** the concentration of the isolated L-threonine or L-tryptophan is determined.

## Revendications

1. Micro-organismes recombinants de la famille des Enterobacteriaceae, produisant de la L-thréonine ou du L-tryptophane, qui contiennent un gène tig surexprimé, qui code pour le Trigger Factor (TF), la souche parente utilisée pour les mesures de la surexpression du gène tig présentant déjà l'aptitude à enrichir au moins 0,25 g/l de L-thréonine ou de L-tryptophane en au maximum 120 heures dans la cellule et/ou dans le milieu nutritif ou le bouillon de fermentation
a) les micro-organismes étant préparés par transformation, par transduction, par conjugaison ou par une combinaison de ces procédés, avec un vecteur qui contient le gène tig, un allèle de ce gène, ou des parties de ce dernier qui présentent l'activité du Trigger Factor TF, et qui contient éventuellement un promoteur ; et/ou
b) dans les micro-organismes, le nombre de copies du gène tig, ou des allèles, est augmenté d'au moins 1 par rapport aux micro-organismes non recombinants pour le gène tig ; et/ou
c) on mute la région de promoteur et de régulation ou le site de liaison ribosomique en amont du gène tig ou on incorpore des cassettes d'expression ou des promoteurs en aval du gène tig pour atteindre l'amplification.

2. Micro-organismes selon la revendication 1, dans lesquels un polynucléotide, correspondant au gène tig, qui code pour un polypeptide, dont la séquence d'acides aminés a une identité d'au moins 90 % avec une séquence d'acides aminés choisie dans le groupe SEQ ID N° 2, SEQ ID N° 4 et SEQ ID N° 6 est surexprimé.

3. Micro-organismes selon la revendication 2, **caractérisés en ce qu'**ils contiennent un polynucléotide isolé correspondant au gène tig, choisi dans le groupe :
a) le polynucléotide, ayant une séquence nucléotidique choisie parmi SEQ ID N° 1, SEQ ID N° 3 ou SEQ ID N° 5 et les séquences nucléotidiques qui leur sont complémentaires ;
b) le polynucléotide, ayant une séquence nucléotidique qui correspond à la SEQ ID N° 1, SEQ ID N° 3 ou SEQ ID N° 5 dans le cadre de la dégénérescence du code génétique ;
c) la séquence polynucléotide, ayant une séquence qui s'hybride dans des conditions stringentes à la séquence complémentaire de la séquence SEQ ID N° 1, SEQ ID N° 3 ou SEQ ID N° 5, les conditions stringentes étant atteintes par une étape de lavage dans laquelle la température s'étend sur une plage de 64 à 68°C et la concentration du sel dans le tampon s'étend sur une plage de 2xSSC à 0,1xSSC ;
d) le polynucléotide, ayant une séquence SEQ ID N° 1, SEQ ID N° 3 ou SEQ ID N° 5, qui contient les mutants sens fonctionnellement neutres,
les polynucléotides codant pour le Trigger Factor TF.

4. Micro-organismes selon la revendication 2, **caractérisés en ce que** le polypeptide possède une séquence d'acides aminés qui a une identité d'au moins 95 % avec une des séquences choisies dans le groupe SEQ ID N° 2, SEQ ID N° 4 et SEQ ID N° 6.

5. Micro-organismes selon la revendication 2, **caractérisés en ce que** le polypeptide a la séquence d'acides aminés qui a une identité de 100 % avec une des séquences choisies dans le groupe SEQ ID N° 2 ou SEQ ID N° 4.

6. Micro-organismes selon la revendication 1, dans lesquels le nombre de copies du gène tig, ou des allèles, est augmenté d'au plus 8, de préférence d'au plus 4.

7. Micro-organismes selon la revendication 6, **caractérisés en ce que** l'augmentation, d'au moins 1, du nombre de copies du gène tig est réalisée par intégration du gène ou des allèles dans le chromosome des micro-organismes.

8. Micro-organismes selon la revendication 6, **caractérisés en ce que** l'augmentation, d'au moins 1, du nombre de copies du gène tig est atteinte par un vecteur à réplication extra-chromosomique.

9. Micro-organismes selon les revendications 1 à 8, **caractérisés en ce que**, sous l'effet de l'amplification du gène tig, la concentration ou l'activité du produit génique tig (protéine) augmente d'au moins 10 %, par rapport à l'activité ou à la concentration du produit génique dans le micro-organisme ou la souche parente non recombinant pour le gène tig.

10. Micro-organismes selon les revendications 1 à 9, **caractérisés en ce que** les micro-organismes sont choisis parmi les genres Escherichia, Erwinia, Providencia et Serratia.

11. Micro-organismes selon les revendications 1 à 10, **caractérisés en ce qu'**ils produisent de la L-thréonine.

12. Procédé de préparation des acides L-aminés L-thréonine ou L-tryptophane par fermentation de micro-organismes recombinants de la famille des Enterobacteriaceae selon les revendications 1 à 11, **caractérisé en ce que**
a) on cultive les micro-organismes produisant de la L-thréonine ou du L-tryptophane qui contiennent un gène tig surexprimé, qui code pour le Trigger Factor TF, dans un milieu dans des conditions dans lesquelles l'acide L-aminé souhaité L-thréonine ou L-tryptophane va subir un enrichissement dans le milieu ou dans les cellules, et
b) on isole l'acide L-aminé souhaité L-thréonine ou L-tryptophane, des constituants additionnels du bouillon de fermentation et/ou la biomasse éventuellement restant en totalité ou en partie (≥ 0 à 100 %) dans le produit isolé, ou étant entièrement éliminés.

13. Procédé selon la revendication 12, **caractérisé en ce que**, pour la préparation de L-thréonine, on fermente des micro-organismes dans lesquels on surexprime, en plus et simultanément un ou plusieurs des gènes choisis dans le groupe consistant en :
a) au moins un gène de l'opéron thrABC, codant pour l'aspartatekinase, l'homosérine-déshydrogénase, l'homosérinekinase et la thréoninesynthase,
b) la gène pyc de Corynebacterium glutamicum, codant pour la pyruvatecarboxylase,
c) le gène pps codant pour la phosphoénolpyruvate-synthase,
d) le gène ppc codant pour la phosphoénolpyruvate-carboxylase,
e) les gènes pntA et pntB codant pour les sous-unités de la pyrdine-transhydrogénase,
f) le gène rhtC codant pour la protéine conférant une résistance à la thréonine,
g) le gène thrE de Corynebacterium glutamicum, codant pour la protéine transporteuse d'exportation de la thréonine,
h) le gène gdhA codant pour la glutamate-déshydrogénase,
i) le gène ptsH codant pour la phosphohistidine-protéine-hexose-phosphotransférase,
j) le gène ptsI codant pour l'enzyme I du système phosphotransférase,
k) le gène crr codant pour le composant IIA spécifique du glucose du système phosphotransférase,
l) le gène ptsG codant pour le composant IIBC spécifique du glucose,
m) le gène cysK codant pour la cystéinesynthase A,
n) le gène cysB codant pour le régulateur du régulon cys,
o) le gène cysJ codant pour la flavoprotéine de la NADPH-sulfite-réductase,
p) le gène cysI codant pour l'hémoprotéine de la NADPH-sulfite-réductase,
q) le gène cysH codant pour l'adénylylsulfate-réductase,
r) le gène sucA codant pour la sous-unité décarboxylase de la 2-cétoglutarate déshydrogénase,
s) le gène sucB codant pour la sous-unité dihydrolipoyltranssuccinase E2 de la 2-cétoglutarate déshydrogénase,
t) le gène sucC codant pour la sous-unité β de la succinyl-CoA synthétase,
u) le gène sucD codant pour la sous-unité α de la succinyl-CoA synthétase,
v) le produit génique du cadre de lecture ouvert (ORF) yjcG d'Escherichia coli,
w) le produit génique du cadre ouvert de lecture (ORF) yibD d'Escherichia coli,
x) le produit génique du cadre ouvert de lecture (ORF) yaaU d'Escherichia coli,
y) le gène secB codant pour le chaperon de protéines exportées SecB du complexe de sécrétion de protéine Sec
z) le gène mopB codant pour le chaperon GroES, régulateur de Hsp60,
aa) le gène dnaK codant pour le chaperon Hsp70 (DnaK)
bb) Le gène dnaJ codant pour le chaperon DnaJ, chaperon partenaire de DnaK,
cc) le gène mopA codant pour le chaperon Hsp60 (GroEL),
dd) le gène ftsZ codant pour la protéine essentielle FtsZ de division cellulaire liant le GTP, et
ee) le gène htpG codant pour le chaperon Hsp90 (htpG).

14. Procédé selon la revendication 12, **caractérisé en ce que**, pour la préparation de L-tryptophane, on fermente des micro-organismes de la famille des Enterobacteriaceae, dans lesquels on surexprime, en plus et simultanément un ou plusieurs des gènes choisis dans le groupe comprenant :
a) au moins un gène de l'opéron tryptophane codant pour l'anthranilate-synthase, l'anthralinate-phosphoribosyl-transférase, la phosphoribosylanthranilate-isomérase, l'indole-3-glycérinephosphate-synthase, et la tryptophane-synthase,
b) le gène serA codant pour la 3-phosphoglycérate-déhydrogénase,
c) le gène serB codant pour la phosphosérine-phosphatase,
d) le gène serC codant pour la phosphosérine-aminotransférase,
e) le gène aroF codant pour la DHAP-synthase sensible à la L-tyrosine,
f) le gène aroG codant pour la DHAP-synthase sensible à la L-phénylalanine,
g) le gène aroH codant pour la DHAP-synthase sensible au L-tryptophane,
h) le gène pps codant pour la phosphoénolpyruvate-synthase,
i) le gène pckA codant pour la phosphoénolpyruvate-carboxykinase,
j) le gène tktA codant pour la transkétolase A,
k) le gène tktB codant pour la transkétolase B, et
l) le produit génique du cadre ouvert de lecture (ORF) yddG d'Escherichia coli,
m) le gène secB codant pour le chaperon de protéines exportées SecB du complexe de sécrétion de protéine Sec
n) le gène mopB codant pour le chaperon GroES, régulateur de Hsp60,
o) le gène dnaK codant pour le chaperon Hsp70 (DnaK)
p) Le gène dnaJ codant pour le chaperon DnaJ, chaperon partenaire de DnaK,
q) le gène mopA codant pour le chaperon Hsp60 (GroEL),
r) le gène ftsZ codant pour la protéine essentielle FtsZ de division cellulaire liant le GTP, et
s) le gène htpG codant pour le chaperon Hsp90 (htpG).

15. Procédé selon les revendications 12 et 13, **caractérisé en ce que**, pour la préparation de L-thréonine, on fermente des micro-organismes dans lesquels on désactive ou on en diminue l'expression, en plus et simultanément un ou plusieurs des gènes choisis dans le groupe consistant en :
a) le gène tdh codant pour la thréonine-déshydrogénase,
b) le gène mdh codant pour la malate-déshydrogénase,
c) le gène pckA codant pour la phosphoénolpyruvate-carboxykinase,
d) le gène poxB codant pour la pyruvate-oxydase,
e) le gène dgsA codant pour le régulateur DgsA du système phosphotransférase,
f) le gène fruR codant pour le répresseur fructose,
g) le gène rpoS codant pour le facteur Sigma³⁸,
h) le gène aspA codant pour l'aspartate ammonium-lyase, et
i) le gène ompF codant pour la porine F de membrane externe.

16. Procédé selon les revendications 12 et 14, **caractérisé en ce que**, pour préparer le L-trytophane, on fermente des micro-organismes de la famille des Enterobacteriaceae, dans lesquels on désactive ou on en diminue l'expression, en plus et simultanément un ou plusieurs des gènes choisis dans le groupe consistant en :
a) le gène tnaA codant pour la tryptophanase,
b) le gène trpR codant pour le répresseur de l'opéron trp,
c) le gène tyrA codant pour la chorismate-mutase T et la préphénate-déshydrogénase,
d) le gène pheA codant pour la chorismate-mutase P et la préphénate-déshydrogénase,
e) le gène mtr codant pour la protéine transporteuse spécifique du tryptophane,
f) le gène tnaB codant pour la tryptophane-perméase,
g) le gène aroP codant pour le transporteur d'acides aminés aromatiques,
h) le gène sdaA codant pour la L-sérine désaminase,
i) le gène pgi codant pour la glucose-6-phosphate-isomérase,
j) le gène tyrB codant pour la tyrosine-aminotransférase, et
k) le gène ompF codant pour la porine F de membrane externe.

17. Procédé selon la revendication 12 à 16, **caractérisé en ce que** les micro-organismes sont cultivés dans un procédé par lots, un procédé à alimentation discontinue, un procédé à alimentation discontinue répétée ou un procédé continu.

18. Procédé selon les revendications 12 à 17, **caractérisé en ce qu'**on détermine la concentration de la L-thréonine ou du L-tryptophane isolé(e).
